# EUROPEAN PATENT APPLICATION

(11) **EP 3 805 373 A1**
(43) Date of publication of application: **14.04.2021**
(21) Application number: 19382889.4
(22) Date of filing: 10.10.2019
(51) Int. Cl.: C12N 5/0793, B82Y 30/00, H01L 51/00

(54) **SUBSTRATES FOR CULTURING AND STIMULATING CELLS**

(71) Applicant: Fundación Imdea Nanociencia, 28049 Madrid (ES); S.I.S.S.A. Scuola Internazionale Superiore di Studi Avanzati, 34136 Trieste (IT)
(72) Inventor: RODRÍGUEZ FERNÁNDEZ, María Isabel, E-28049 Madrid (ES); GONZÁLEZ PÉREZ, María Teresa, E-28049 Madrid (ES); HERNÁNDEZ RUEDA, Jaime Javier, E-28049 Madrid (ES); PÉREZ GARCÍA, Lucas, E-28049 Madrid (ES); CAMARERO DE DIEGO, Julio, E-28049 Madrid (ES); MIRANDA SORIANO, Rodolfo, E-28049 Madrid (ES); BALLERINI, Laura, I-34123 Trieste (IT); CALARESU, Ivo, I-34123 Trieste (IT); RAUTI, Rossana, Tel Aviv (IL); SCAINI, Denis, I-34132 Trieste (IT)
(74) Representative: ABG Intellectual Property Law, S.L.

(57) **Abstract**

The present invention relates to the use of a substrate comprising a textured surface with a plurality of protrusions and a bulk portion, for promoting neural cell network growth in vitro and/or ex-vivo; for a culturing method; and for complex neural cell network reconstruction. The invention also relates to the use of a substrate for cell stimulation; and to a probe, a stimulation device, and a monitor device comprising the substrate and their uses. In addition, the invention relates to compositions comprising the substrate and their medical uses. Moreover the invention relates to a kit comprising the substrate.

## Description

### FIELD OF THE ART

The present invention relates to the use of a substrate comprising a textured surface with a plurality of protrusions for neural cell network growing and for stimulating cells.

### STATE OF THE ART

Nowadays, the wide use of polymeric materials is attributed to the combination of factors such as low cost, low weight and ease of processing. Additional or improved properties in terms of mechanical, thermal, optical or electrical behavior can be imparted onto polymeric materials by incorporation of filler particles to form polymer-based substrates. Micro and/or nano engineered topographies of specific geometrical features or textures can also provide a range of properties or functions in addition to the ones inherent in a material. For example, WO2017167909A1 discloses a composite having a textured surface with a plurality of protrusions and a bulk portion, which comprises a polymeric matrix and particles, being the major amount of particles concentrated into the textured surface and its uses as structural coating, super-repellent material, antireflective material, or antimicrobial material.

Documents in the art suggest the use of polymer substrates with engineered topographies to achieve different functionalities. US20060183222 discloses a method for controlling the morphological growth of neurons providing a culture surface with a plurality of protrusions. Viela, F. *et al.* (Viela, F. et al. Adv. Func. Mater. 2016) used polycarbonate pillared substrates with topographies in the micro and nanoscale, for seeding and culturing neuronal stem cells *in vitro.* Viela, F. et al. disclosed that the morphology, rate of proliferation and migration ability of the cells cultured on nano and microstructured substrates change with topographies at different scales. However, further improvement is necessary to manufacture multifunctional engineered topographies with enhanced properties for growing active cultured cells. In interfacing neuronal growth and function, 2D cultures are limited tools when investigating the role of circuit topology on networks dynamics, for example in "real life" neuronal branches are exposed to the 3-dimensional space. Thus the architecture of neural interfaces influences the tissue re-growth within the platform and the ability of detecting electrical patterned activity from specific brain (cultured) circuitries.

Polymer substrates with engineered topographies are used in prosthetic devices such as cell stimulating and/or recording devices. Advances in material research are crucial in these efforts and new developments are drawing from engineering technologies. For example, Aurand et al. (Adv. Funct. Mater. 2017, 1700550) developed a microporous, self-standing, interface made of polydimethylsiloxane (PDMS) comprising carbon nanotubes and use it as a substrate to growth a synchronized neural network from spinal explants in vitro. In addition, the biocompatibility of said substrate was assessed in vivo. The elastomeric platform developed by Aurand et al. has several limitations: first, the manufacturing of reproducible scaffolds requires a highly specialized and dedicated material and chemical engineering laboratory, making unfeasible an industrial and cost effective upscale of platforms of standard dimensions or mechanical properties. Second, in Aurand et al. when tested in vivo, in the absence of nanotubes, the elastomeric scaffolds did not adhere to the tissue, leading to an encapsulation of the device which would not favor a regenerative interface stable implant; in addition the bulk material being electrically insulating, to control path of conductivity for effective delivery and/or recording of electrical signals would be, on a large scale production demand, very unlikely.

In addition, there is a need in the art for tailored and multifunctional polymer substrates having further functionalities, in particular, the ability to interact with a cell culture or tissue and improved adaptability to the cell culture or tissue.

### BRIEF DESCRIPTION OF THE INVENTION

The object of the present invention is to develop a cost-effective, easy to use substrate that will promote cell growth and interfacing within a cell or tissue culture, in particular guiding network growth.

In contrast with the substrates known in the art, the use of the substrate of the invention wherein it's textured surface has a plurality of protrusions, it presents improved interaction with cells and allows the growing of active cell cultures on it. Moreover the use of the substrate of the invention, in particular its textured surface, provide cultured cells with mechanical cues and play an important role to direct cellular functions and tissue organization. Of particular relevance is the ability of the substrate to provide a three dimension microenvironment for axons to navigate, allowing the reconstruction of 3D topologies, that is the way neurons are synaptically connected in a given network. Such a property will be exploited for neuronal growth, recording and stimulation. In addition, the substrate of the present invention is biocompatible.

In addition, the use of the substrate of the invention wherein both the textured surface and/or the bulk portion comprise particles having a mean size of between 1 nm and 1000 µm; wherein said particles are homogeneously distributed among the bulk portion and the textured surface; and wherein the particles are electrically conductive, presents improved electrical properties of the substrates of the art. In addition, it has an improved biocompatibility than metallic interfaces and reduces gliosis and scar formation.

Therefore, in a first aspect, the invention is directed to the use of a substrate comprising:
a) a textured surface with a plurality of protrusions, and
b) a bulk portion;
wherein both the textured surface and/or the bulk portion comprise a polymeric matrix; for promoting active neural cell network growth *in vitro* and/or *ex-vivo;* preferably for promoting active neural cell network 3D growth *in vitro* and/or *ex-vivo.*

In a second aspect, the invention is directed to an *in vitro* and/or *ex-vivo* culturing method comprising the steps:
i) providing
   a) the substrate as defined in claim 1; and
   b) primary cultures developed from dissociated brain cells and organotypic cultures from explanted neural tissue; wherein the primary cultures comprise cells;
   wherein the substrate and the cells are in an *in vitro* and/or *ex-vivo* culture media; and wherein the textured surface with a plurality of protrusions of the substrate (a) is in contact with the surface of one or several cells (b) in the culture media;
ii) culturing the cells to obtain a neural cell network; and
iii) measuring the neural cell network activity.

In a third aspect, the invention is directed to a cultured neural cell network obtainable by the culturing method of the present invention; wherein the cultured neural cell network is active.

In a further aspect, the invention is directed to the use of a substrate comprising:
a) a textured surface with a plurality of protrusions, and
b) a bulk portion;
wherein both the textured surface and/or the bulk portion comprise a polymeric matrix;
- wherein both the textured surface and/or the bulk portion comprise particles having a mean size of between 1 nm and 1000 µm,
- wherein said particles are homogeneously distributed among the bulk portion and the textured surface, and
- wherein the particles are electrically conductive,
for promoting neural network growth *in vitro* and/or *ex-vivo* and/or stimulation *in vitro* and/or *ex*-*vivo*; preferably for promoting neural cell network 3D growth *in vitro* and/or *ex-vivo* and/or stimulation of electrically excitable cells *in vitro* and/or *ex-vivo.*

In a further aspect, the invention is directed to a probe comprising:
- a substrate comprising:
   a) a textured surface with a plurality of protrusions, and
   b) a bulk portion;
      - wherein both the textured surface and/or the bulk portion comprise a polymeric matrix;
      - wherein both the textured surface and/or the bulk portion comprise particles having a mean size of between 1 nm and 1000 µm,
      - wherein said particles are homogeneously distributed among the bulk portion and the textured surface, and
      - wherein the particles are electrically conductive; and
- an electrical connection comprising a conductive wire;
wherein the probe is optionally implantable.

In another aspect, the invention is directed to the use of the probe of the present invention for cell stimulation *in vitro* and/or *ex-vivo.*

In another aspect, the invention is directed to a stimulation device comprising
- the probe as defined in the present invention; and
- an electrical pulse generator;
wherein the probe and the electrical pulse generator are electrically connected; and wherein the device is optionally implantable.

In yet another aspect, the invention is directed to the use of the stimulation device of the present invention for *in vitro* and/or *ex-vivo* cell stimulation and/or cell neuromodulation.

In another aspect, the invention is directed to a monitor device comprising
- the probe as defined in the present invention or the stimulation device of the present invention; and
- an electrical monitor;
wherein the probe and the electrical monitor are electrically connected.

In yet another aspect, the invention is directed to the use of the monitor device for cell monitoring *in vitro* and/or *ex-vivo.*

In another aspect, the invention is directed to a composition obtainable by the method comprising the steps of:
- providing
   ∘ the probe or the stimulation device or the monitor device of the present invention; and
   ∘ one or several electrically excitable cells in an *in vitro* and/or *ex-vivo* media;
- contacting the textured surface of the substrate of the probe with the surface of one or several electrically excitable cells; and
- electrically stimulating the one or several electrically excitable cells to obtain a composition.

In another aspect, the invention is directed to a composition comprising:
- the probe or the stimulation device or the monitor device of the present invention; and
- electrically excitable cells;
wherein the textured surface of the substrate of the probe is in contact with the surface of one or several cells in an *in vitro* and/or *ex-vivo media.*

In addition, the present invention is directed to any of the compositions of the present invention for use as a medicament.

The present invention is directed to the compositions of the present invention for use in the treatment of chronic pain, epilepsy, and Parkinson's disease, sacral nerve pelvic disorders or incontinence, spinal cord traumatic disorders and spinal cord ischemic disorders (such as angina or peripheral vascular disease).

Finally, another aspect of the present invention is directed to a kit comprising
- the probe or the stimulation device or the monitor device of the present invention;
- electrically excitable cells; and
- at least a container;
wherein the textured surface of the substrate of the probe is in contact with the surface of one or several cells in an *in vitro* and/or *ex-vivo media.*

### FIGURES

The accompanying figures illustrate exemplary embodiments of the invention and, together with the written description serve to explain the principles of the invention.

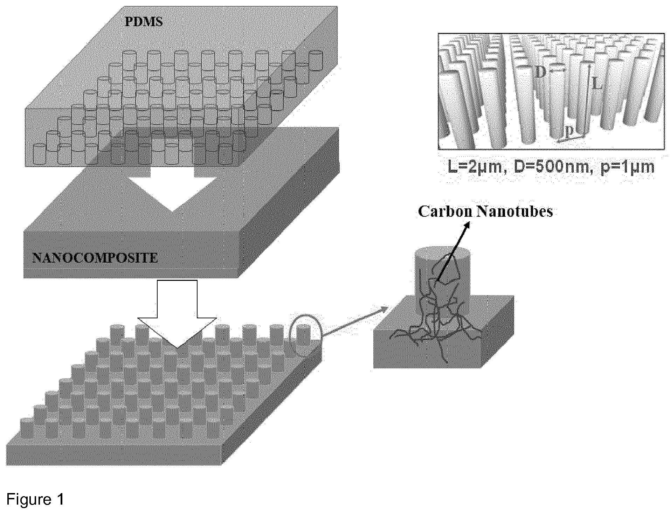

Figure 1: Outline of the fabrication process of nanostructured conductive PS-SWCNT substrates.

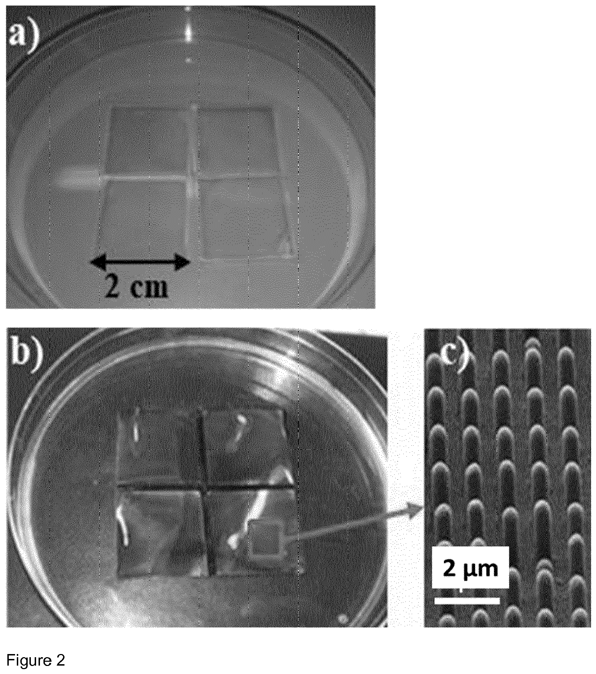

Figure 2: Images of the nanostructured substrates fabricated in (a) polystyrene (PS) and (b) PS-SWCNT nanostructured substrates. (c) Scanning electron microscopy (SEM) detail of the 500 nm pillars array of the nanostructured substrates.

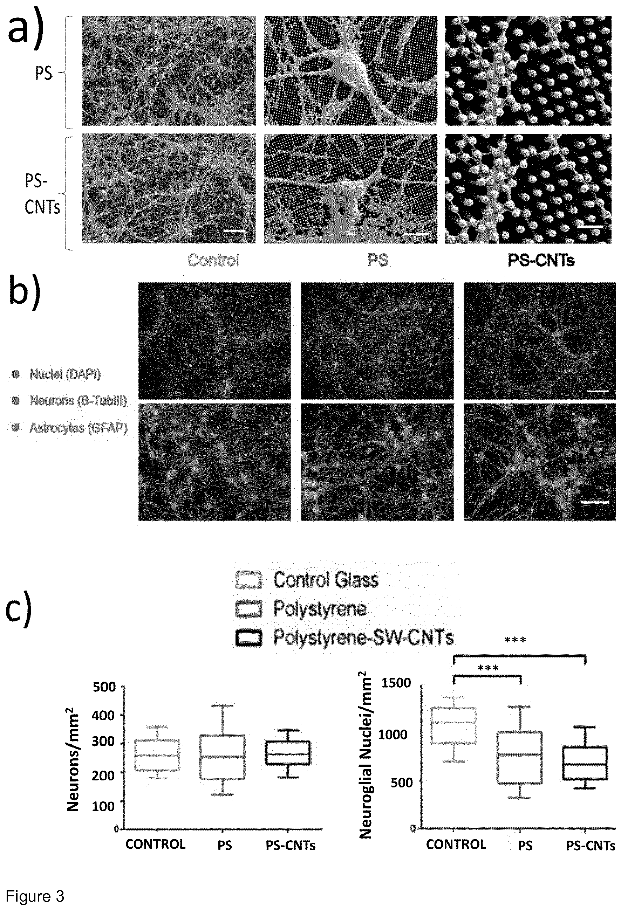

Figure 3: Cultured brain cell densities on glass flat substrate (control) and PS and PS-CNTs nanostructured substrates (a) Scanning Electron Microscopy (SEM) micrographs of neuronal cultures grown onto PS and PS-CNTs nanostructured substrates. Scale bars = 40 µm (left), 10 µm (middle) and 2 µm (right). Figure 3 (b) shows fluorescence micrographs of neural cell network cultures with DAPI and markers of specific cytoskeletal components (neurons and astrocytes). Figure 3 (c) box-plots showing neuronal densities (neurons/mm²) and neuroglial nuclei density (neuroglial nuclei/mm²) of neuronal cultures grown onto a glass flat substrate (control) and PS and PS-CNTs nanostructured substrates.

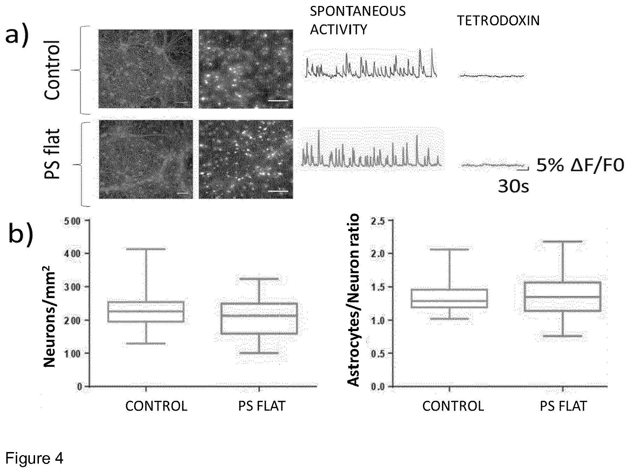

Figure 4: Cultured neurons morphology and activity on poly-L-ornithine coated flat glass (control) and flat polystyrene surfaces (PS-flat) (a) From left to right: a representative cultures visualized by immunostaining (scale bar = 50 µm); a representative snapshots of Oregon green-loaded neurons (scale bar = 100 µm) visualizing a live imaging recording field and their corresponding spontaneous activity recorded as Ca²⁺ fluorescence fluctuations over time expressed as fractional amplitude increase (ΔF/F0) prior and after the addition of tetrodotoxin; and (b) neuronal (neurons/mm²) and astrocytes densities (astrocytes neuron/mm²) in poly-L-ornithine control and PS-flat.

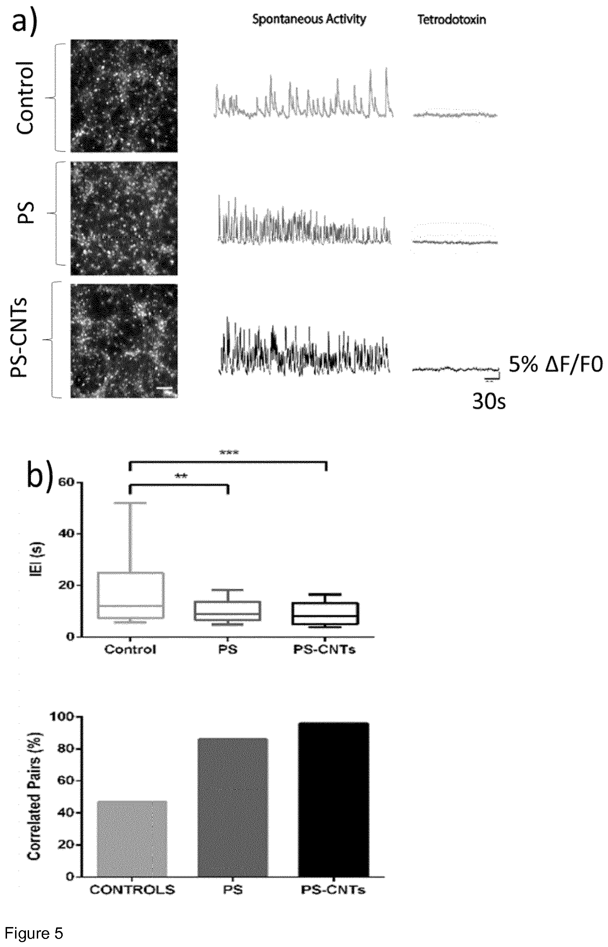

Figure 5: Functional networks on glass flat substrate (control) and PS and PS-CNTs nanostructured substrates. (a) Left: snapshots of Oregon green-loaded neural cell cultures and their corresponding spontaneous activity as Ca²⁺ fluorescence recorded over time expressed as fractional amplitude increase (ΔF/F0) prior and after the addition of tetrodotoxin measured from cultures grown onto poly-L-ornithine coated flat glass (control), and PS, PS-CNTs nanostructured substrates. Scale bar = 100 µm. (b) top, box-plots of the inter-event intervals (IEI (s)) from cell cultures grown onto control flat substrate and PS and PS-CNTs nanostructured substrates; bottom, bar plot reporting the number of correlated pairs to measure of synchronous activity of neurons, wherein each pair represent the spontaneous activity of two non-neighboring cultured cells grown onto control, PS and PS-CNT nanostructured substrates, expressed as percentage of the total amount of cells pair analyzed.

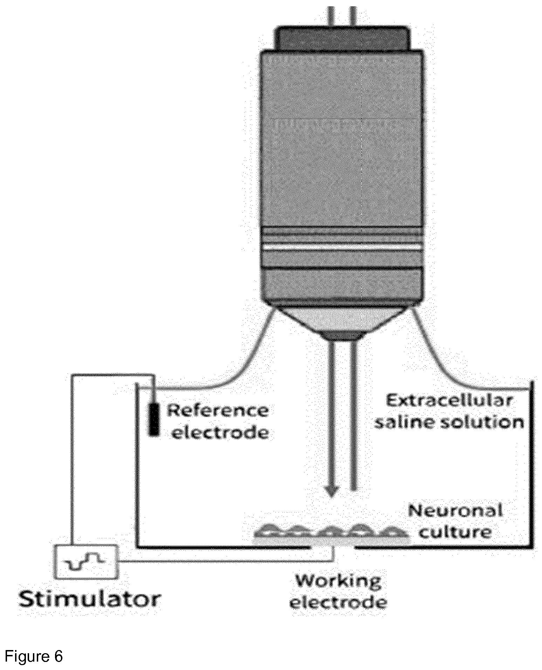

Figure 6: sketch of the experimental set up used for electrically stimulating neural cultured networks grown onto PS-CNT nanostructured substrates.

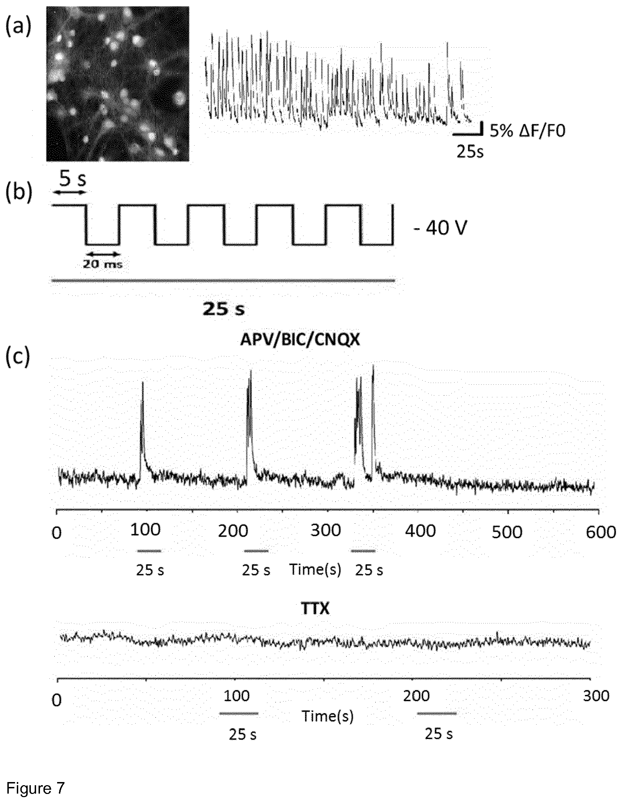

Figure 7: Live imaging of stimulated neurons on PS-CNTs (a) left: snapshot of a representative field of neural cultures grown onto a PS-CNT nanostructured substrate labeled by Oregon green for calcium live imaging; right: recording of spontaneous synaptic activity measured as live calcium fluorescence changes recorded over time as fractional amplitude increase (ΔF/F0) in the absence of stimulation and of receptors blockers; (b) electrical stimulation protocol applied to neural cultures grown onto PS-CNT nanostructured substrates (electrical pulses train); (c) synaptic activity measured as live calcium fluorescence imaging recorded over time as fractional amplitude increase (ΔF/F0) in the presence of pharmacological blockers of synaptic receptors (glutamate NMDARs by APV 25 µM; GABA_{A}R by bicuculline 10 µM and glutamate AMPARs by CNQX 10 µM) to functionally de-couple the recorded cells from the network, here a single neuron is depicted for clarity, where evoked activity was repeatedly induced.

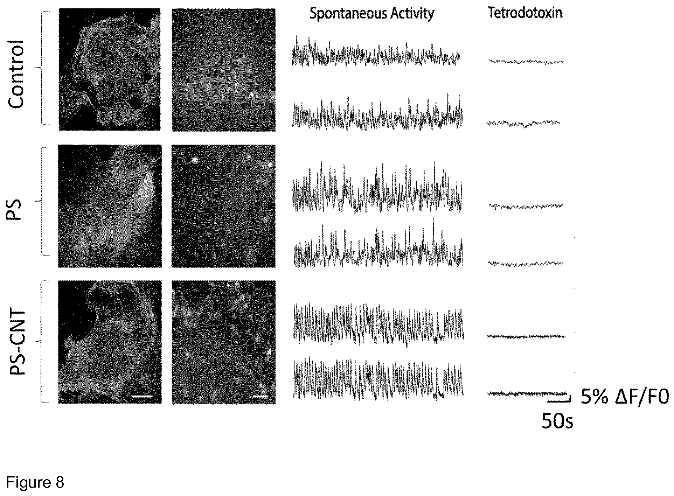

Figure 8: Biocompatibility of the substrates for tissue explants growth ex-vivo Left: confocal reconstructions of immunofluorescence images of organotypic spinal cord cultures onto poly-L-ornithine coated flat glass (control), PS and PS-CNTs nanostructured substrates; scale bar = 500 µm. Middle: snapshots of 3 representative fields of the ventral horn in slice cultures labeled with calcium indicator Fluo-4 AM and right: representative traces for spontaneous synaptic activity measured as live calcium fluorescence imaging recorded over time as fractional amplitude increase (ΔF/F0) in the absence and presence of tetrodotoxin.

### DETAILED DESCRIPTION OF THE INVENTION

### Substrate

In the context of the present invention, the term "substrate" is understood as comprising:
a) a textured surface with a plurality of protrusions, and
b) a bulk portion;
wherein both the textured surface and/or the bulk portion comprise a polymeric matrix.

The term "textured surface" in the context of the present invention, refers to the surface of the substrate of the invention having a plurality of micro- and/or nanometric protrusions. The protrusions in the substrate of the invention may have any common regular and/or irregular shapes, such as a spherical shape, elliptical shape, cubical shape, tetrahedral shape, pyramidal shape, octahedral shape, cylindrical shape, polygonal pillar-like shape, conical shape, columnar shape, tubular shape, helical shape, funnel shape, or dendritic shape. Each of the protrusions may have the same or different shape, height, and/or width. In a particular embodiment, the protrusions have a columnar shape or a cylindrical shape. In another particular embodiment, the protrusions have a pillar shape. If the protrusions are in the nanometric range, the textured surface may be referred as nanostructured surface.

In one embodiment, the protrusions in the textured surface of the substrate of the invention present a height of between 5 nm and 1000 µm, preferably between 5 nm and 100 µm, more preferably of between 10 nm and 20 µm, more preferably between 50 nm and 10 µm, more preferably of between 100 nm and 5 µm, even more preferably between 200 nm and 3 µm, even more preferably between 250 and 2000 nm. In another preferred embodiment, the protrusions in the textured surface present a height of between 1 µm and 12 µm.

In one embodiment, the protrusions in the textured surface of the substrate of the invention present a width between 5 nm and 1000 µm, preferably between 5 nm and 100 µm, more preferably between 10 nm and 20 µm, more preferably of between 50 nm and 10 µm, more preferably of between 10 nm and 5 µm, even more preferably between 200 nm and 1 µm, even more preferably between 250 and 800 nm, even much more preferably around 500 nm. In another preferred embodiment, the protrusions in the textured surface present a width of between 1 µm and 2 µm. In the context of the present invention, the expression width may be considered as diameter.

In one embodiment, the protrusions in the textured surface of the substrate of the invention present an interval between the protrusions or pitch of between 5 nm and 1000 µm, preferably between 5 nm and 100 µm, more preferably of between 10 nm and 20 µm, more preferably of between 50 nm and 10 µm, more preferably of between 100 nm and 5 µm, even more preferably between 200 nm and 2 µm, even more preferably between 250 and 1.5 µm. In another preferred embodiment, the protrusions in the textured surface present an interval of between 1 µm and 4 µm. The intervals between the protrusions may also be the same or different.

In a particular embodiment, the substrate of the invention comprises a textured surface with a plurality of protrusions, wherein the protrusions in the textured surface present a height of between 50 nm and 10 µm, a width of between 50 nm and 1000 nm, and an interval between the protrusions from 50 nm to 2000 nm. In another particular embodiment, the protrusions in the textured surface of the substrate of the invention present a height of 2 µm, a width of 0.5 µm and an interval between protrusions of 1 µm.

In a particular embodiment, the term "comprise" in the expression "both the textured surface and/or the bulk portion comprise a polymeric matrix" may be interpreted as an open term.

In a particular embodiment, the substrate of the present invention comprises:
a) a textured surface with a plurality of protrusions, and
b) a bulk portion;
wherein both the textured surface and/or the bulk portion consist of a polymeric matrix.

In a particular embodiment, the polymer in the polymeric matrix is selected from thermosetting polymers or thermoplastic polymers.

Thermosetting polymers may be selected from polyester resins or epoxy resins. Polymers may also be selected from unsaturated rubbers such as natural polyisoprene; natural rubber (NR) and gutta-percha, synthetic polyisoprene, chloroprene rubber (CR), neoprene, butyl rubber and halogenated butyl rubbers styrene-butadiene rubber, nitrile rubber and hydrogenated nitrile rubbers; or saturated rubbers such as ethylene propylene rubber, ethylene propylene diene rubber, epichlorohydrin rubber (ECO), polyacrylic rubber (ACM, ABR), silicone rubber, fluorosilicone rubber (FVMQ), fluoroelastomers, tecnoflon, perfluoroelastomers, polyether block amides (PEBA), chlorosulfonated polyethylene (CSM), from polyurethanes elastomers, or from silicones elastomers (such as siloxanes like polydimethylsiloxane (PDMS)). Preferably, the thermosetting polymer is PDMS.

Thermoplastic polymers may be selected from thermoplastic polyurethane (TPU), polyolefins (such as polyethylene (PE), polypropylene (PP), poly(tetrafluoroethylene) (PTFE)), acrylates (such as poly(methyl acrylate) (PMMA)), polyethers such as (polyether ether ketone (PEEK), polyether sulfone (PES)), polystyrene (PS), polyamides (PA). Preferably, the thermoplastic polymer is PS.

In a more particular embodiment, the polymer in the polymeric matrix of the substrate of the invention is a thermoplastic polymer. In a particular embodiment, the thermoplastic polymer is selected from poly(methyl methacrylate) (PMMA), polycarbonate (PC), polystyrene (PS),cellulose acetate (CA) and mixtures thereof. In another particular embodiment the thermoplastic polymer is a polyolefin selected from polypropylene (PP), polyethylene (PE), and mixtures thereof. In another particular embodiment the thermoplastic polymer is selected from polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polytrimethylene terephthalate (PTT), polyethylene naphthalate (PEN) and mixtures thereof. In a preferred embodiment the thermoplastic polymer is selected from polyglycolide (PGA), poly(propylenefumarate) (PPF), polycyanoacrylate (PCA), polycaprolactone (PCL), poly(glycerol sebacate) (PGS), poly(glycerol sebacate acrylate) (PGSA), polyvinylidenefuoride (PVDF), polyvinylidene chloride (PVDC), polyethylene terephthalate (PET), polybutylene therephtalate (PBT), polyphenylene oxide (PPO), polyvinyl chloride (PVC), cellulose acetate, cyclic olefin copolymer (COC), ethylene vinyl acetate (EVA), ethylene vinyl alcohol, (EVOH), polyethersulfone (PES), chlorinated poly ethylene (CPE), poly 3-hydroxybutyrate (P3HB), polybutylene adipate (PBA), polyethylene adipate (PEA), polybutylene succinate (PBS), polyphenylene sulfide (PPS), polysulfone (PSU), polytrimethylene terephthalate (PTT), polyurethane (PU), polyvinyl acetate (PVAc), styrene acrylonitrile (SAN), and mixtures thereof. In another particular embodiment the thermoplastic polymer is selected from polyetherketones (PEEK), polyvinylalcohol (PVA), polyhydroxyethylmethacrylate polyvinylalcohol (PHMM-PVA), polyhydroxyethylmethacrylate (PHEMA), poly(N-isopropylacrylamide) (PNIPAAm) and mixtures thereof. In a preferred embodiment the polymer in the polymeric matrix is polystyrene (PS), polycarbonate (PC), polyethylene (PE), polypropylene (PP), poly(methyl methacrylate) (PMMA), polydimethylsiloxane (PDMS) and mixtures thereof; preferably polydimethylsiloxane (PDMS) or polystyrene (PS); more preferably polystyrene (PS).

In a preferred embodiment, the thermoplastic polymer is selected from poly(methyl methacrylate) (PMMA), polycarbonate (PC), polystyrene (PS), polypropylene (PP), polyethylene (PE), polyglycolide (PGA), poly(propylenefumarate) (PPF), polycyanoacrylate (PCA), polycaprolactone (PCL), poly(glycerol sebacate) (PGS), poly(glycerol sebacate acrylate) (PGSA), polyvinylidenefuoride (PVDF), polyvinylidene chloride (PVDC), polyethylene terephthalate (PET), polybutylene therephtalate (PBT), polyphenylene oxide (PPO), polyvinyl chloride (PVC), cellulose acetate (CA), cyclic olefin copolymer (COC), ethylene vinyl acetate (EVA), ethylene vinyl alcohol, (EVOH), polytetrafluoroethylene (PTFE), fluorinated ethylene propylene (FEP), perfluoroalkoxy (PEA), ethylene tetrafluoroethylene (ETFE), polyethersulfone (PES), chlorinated poly ethylene (CPE), polylactic acid (PLA), poly 3-hydroxybutyrate (P3HB), polybutylene adipate (PBA), polyethylene adipate (PEA), polybutylene succinate (PBS), polyphenylene sulfide (PPS), polysulfone (PSU), polytrimethylene terephthalate (PTT), polyurethane (PU), polyvinyl acetate (PVA), polyvinylidene chloride (PVDC), styrene acrylonitrile (SAN), polyetherketones (PEEK), polyvinylalcohol (PVA), polyhydroxyethylmethacrylate (PHEMA), poly(N-isopropylacrylamide) (PNIPAAm) and mixtures thereof.

The substrate of the invention can be used as a free-standing material, or adhered onto a surface, or coated on a surface of a product; preferably coated on a surface of an electrode.

In another particular embodiment, the substrate of the invention is part of a device or a kit; preferably part of a medical device of a medical kit.

### Substrate comprising particles

In a particular embodiment, the substrate of the present invention comprises a filler comprising particles; preferably conductive particles; more preferably electrically conductive particles.

In a preferred embodiment, the substrate of the present invention comprises:
a) a textured surface with a plurality of protrusions, and
b) a bulk portion;
wherein both the textured surface and/or the bulk portion comprise a polymeric matrix;
wherein both the textured surface and/or the bulk portion of the substrate of the present invention comprise particles having a mean size of between 1 nm and 1000 µm; wherein said particles are distributed among the bulk portion and the textured surface, and wherein the particles are preferably conductive particles, more preferably electrically conductive particles.

In a preferred embodiment, the particles of the substrate of the present invention are distributed in the polymeric matrix; preferably homogeneously distributed in the polymeric matrix among the bulk portion and the textured surface of the substrate of the present invention.

In a preferred embodiment, the particles of the substrate of the present invention are embedded in the polymeric matrix; preferably homogeneously embedded and/or distributed in the polymeric matrix.

In the context of the present invention, the term "homogeneously" is related with the particle distribution in the polymeric matrix of the present invention, in particular, it means that the particles are well distributed in the polymeric matrix.

In a particular embodiment, the substrate comprises between 0.1 and 80 wt% of particles; preferably between 0.1 and 40 wt% of particles; more preferably between 0.1 and 30 wt%; even more preferably between a 0.1 and a 5 wt%.

In another particular embodiment, the substrate comprises between 0.01 and 1 g/cm³ of particles; preferably between 0.05 and 0.5 g/cm³ of particles.

In another particular embodiment, the substrate comprises between 0.5 and 1000 particles/cm³, preferably between 1 and 100 particles/cm³.

In a particular embodiment, the particles of the substrate of the invention present a mean size of between 1 nm and 1000 µm. In the context of the present invention by the term "mean size" it is understood the average size of the largest dimension of each particle of the substrate of the invention. The mean size of the particles of the present invention may be calculated from the size values of a statistically significant sample measured by methods known in the art, for example by electrical microscopy. Preferably, the particles of the substrate of the invention present a mean size of between 2 nm and 100 µm, more preferably, between 10 nm and 50 µm, more preferably between 20 nm and 30 µm, even more preferably from 10 nm and 15 µm. In a preferred embodiment, the particles of the substrate of the invention present a mean size of between 100 nm and 10 µm, more preferably of between 200 nm and 5 µm, more preferably of between 300 nm and 3 µm, even more preferably of between 500 nm and 2 µm. In a more preferred embodiment, the particles of the substrate of the invention present a mean size of between 700 nm and 1 µm, preferably between 800 nm and 900 nm. In another preferred embodiment, the particles of the substrate of the invention present a mean size of between 1 nm and 1 µm, preferably between 10 nm and 900 nm.

The particles of the substrate of the invention may have any common regular and/or irregular shape, such as a spherical shape, elliptical shape, cubical shape, tetrahedral shape, pyramidal shape, octahedral shape, cylindrical shape, polygonal pillar-like shape, conical shape, columnar shape, tubular shape, helical shape, funnel shape, wire shape or dendritic shape. In addition, a mixture or particles with different shapes may be also used, for example tubular and spherically shaped particles.

In a particular embodiment, the substrate of the present invention is electrically conductive. In a more particular embodiment, the substrate of the present invention is able to pass/transmit electrical current with applied voltage either through faradic or capacitive mechanisms and either through its textured surface or through the bulk portion. Preferably, the textured surface is typically in electrical contact with the bulk portion and with the surface of the base of the substrate.

In a particular embodiment, the particles in the substrate of the invention are selected from carbon based particles, metal particles, and mixtures thereof.

In another particular embodiment the particles in the substrate of the invention are carbon based particles selected from carbon nanotubes, carbon nanowires, reduced graphene oxide flakes (RGO), carbon nanoribbons, carbon nanorods, carbon nanodots, fullerenes, graphene flakes, carbon black, graphite, carbon nanofibers and mixtures thereof. Preferably, the particles in the substrate of the invention are carbon nanotubes, carbon nanofibers, reduced graphene oxide flakes (RGO) or mixtures thereof. More preferably, the particles in the substrate of the invention are carbon nanotubes.

In another particular embodiment, the particles in the substrate of the invention are elongated structures selected from carbon nanofibers, carbon nanotubes, carbon nanoribbons, carbon nanorods, carbon nanowires and mixtures thereof.

In a particular embodiment, the particles of the substrate of the invention have a mean sized of between 1 nm and 1000 µm; more preferably between 1 nm and 100 µm; even more preferably between 1 nm and 1 µm.

In another particular embodiment, the particles of the substrate of the invention are elongated structures having a mean size of between 0.5 µm and 100 µm, preferably of between wherein said mean size corresponds to the length of the elongated structure. Preferably, the particles of the substrate of the invention are elongated structures having a mean size of between 0.5 µm and 80 µm, preferably of between 2 µm and 50 µm, more preferably of between 10 µm and 30 µm, even more preferably of between 15 µm and 25 µm.

In a preferred embodiment, the particles in the substrate of the invention are carbon nanotubes (CNTs). Preferably, the particles in the substrate of the invention are single walled carbon nanotubes (SWCNT), multiwalled carbon nanotubes (MWCNT) or mixtures thereof.

In a preferred embodiment, the particles in the substrate of the invention are single walled carbon nanotubes (SWCNT) having an average diameter of between 0.5 to 3 nm. In a more preferred embodiment, the particles in the substrate of the invention are single walled carbon nanotubes (SWCNT) having an average diameter of between 0.5 to 3 nm and a length of between 0.5 µm and 100 µm, preferably a length of between 2 to 30 µm.

In another preferred embodiment, the particles in the substrate of the invention are multiwalled carbon nanotubes (MWCNT) having an average diameter of between 4 to 40 nm, preferably of between 10 to 30 nm. In a more preferred embodiment, the particles in the substrate of the invention are multiwalled carbon nanotubes (MWCNT) having an average diameter of between 4 to 40 nm of diameter, preferably of between 10 to 30 nm of diameter and a length of between 0.5 µm and 1000 µm, preferably a length of between 2 to 30 µm.

In another particular embodiment, the particles in the substrate are metal particles such as silver (Ag), aluminum (Al), gold (Au), bismuth (Bi), cobalt (Co), chrome (Cr), copper (Cu), iron (Fe), germanium (Ge), indium (In), iridium (Ir), molybdenum (Mo), neodymium (Nd), nickel (Ni), palladium (Pd), platinum (Pt), rhodium (Rh), ruthenium (Ru), silicon (Si), tin (Sn), tantalum (Ta), titanium (Ti), tungsten (W), zinc (Zn), and their alloys.

In a particular embodiment, the particles in the substrate of the invention are metal particles with sizes ranging from 1 to 200 nm, preferably with sizes ranging from 5 to 100 nm, more preferably from 10 to 50 nm.

The particles in the substrate of the invention may have the surface unmodified (pure) or have the surface modified by ligand-coated or ligand bound molecules.

In a preferred embodiment, the substrate of the present invention comprises:
a) a textured surface with a plurality of protrusions, and
b) a bulk portion;
wherein both the textured surface and/or the bulk portion comprise a polymeric matrix,
wherein both the textured surface and/or the bulk portion of the substrate of the present invention comprise carbon nanoparticles having a mean size of between 1 nm and 1000 µm; wherein said carbon nanoparticles are distributed among the bulk portion and the textured surface; preferably wherein the carbon nanoparticles are between 0.1 and 5 wt%, more preferably wherein the carbon nanoparticles are carbon nanotubes; wherein the substrate is conductive. In a more particular embodiment, the textured surface comprises pillars; preferably arrays of pillars.

### Use for interfacing tissue growth

The present invention is directed to the use of the substrate of the present invention as described in any of its embodiments for promoting active neural cell network growth *in vitro* and/or *ex-vivo;* preferably promoting active neural cell network 3D growth *in vitro* and/or *ex-vivo.* It has been observed that the substrate of the present invention may act as an interface for promoting neural cell network growth; preferably for promoting active neural cell network growth; preferably for promoting active neural cell network 3D growth; even more preferably for active neural tissue growth.

In a particular embodiment, the invention is directed to the use of a substrate comprising:
a) a textured surface with a plurality of protrusions, and
b) a bulk portion;
wherein both the textured surface and/or the bulk portion comprise a polymeric matrix; for promoting active neural cell network growth *in vitro* and/or *ex-vivo.*

In the context of the present invention, the term "neural cell" is meant to include cells from the central nervous system (CNS) or the peripheral nervous system (PNS). Exemplary neural cells of the CNS are found in the gray matter of the spinal cord or the brain and exemplary neural cells of the PNS are found in the dorsal root ganglia. The expression "neural cell network" or "neural network" is understood as comprising neural circuits, i.e. a population of neurons interconnected by synapses; spontaneously active neural cell network, displaying spontaneous electrical and synaptic activity. Thus, synchronized patterned activity emerges when the efficiency of the network connections, synapses, is high.

The "activity" of the neural cell network of the present invention may be measured by calcium imaging characterization techniques known in the art, i.e. by loading the neural cell network with Ca²⁺ dye and imaging the intracellular calcium activity of the cell network; preferably using fluorescence live imaging. In addition, the "activity" of the neural cell network may be identified as from neuronal origin by removing the action potentials using drugs (for example by using tetrodotoxin [TTX]).

In a particular embodiment, the term "activity" refers to a spontaneous and/or not spontaneous activity of the neural cell network as known in the art; preferably to spontaneous activity.

In the context of the present invention, the expressions *"in vitro"* and/or *"ex-vivo"* are understood as known in the art.

As used herein, the expression "growth" regarding cells and/or axons, refers to cell culturing and/or tissue regeneration *in vitro* or ex vivo, i.e. cell and axon grown, spreading and synapse formation, under controlled conditions (such as in a culture media) generally outside their natural environment; in particular to cell culturing on the substrate of the present invention and refers to the ability of a substrate to enhance the growth, spreading and axonal navigation to their target of a cell culture integrated with said substrate; preferably of neural cells; more preferably of neural cell network.

In a particular embodiment, the invention is directed to the use for promoting active neural cell network three dimensional growth; preferably 3D growth.

Without being bound to any theory in particular, the authors of the present invention observed that the neural cell network grown on the substrate of the present invention is healthy, has a regenerative electrical capability, and is able to develop a neuroglia/neuronal balanced condition without adding drugs to prevent extra glial development. In addition, the authors of the present invention observed that an increase in the neural cell network activity was induced by the substrate of the present invention, characterized by an increased efficiency in synchronizing the network, due to the network topology, which strengthens the efficiency of neuronal connectivity. Moreover, the authors of the present invention observed a prominent process branching (axons and dendrites) in the neural cell network grown on the substrate of the present invention. In addition, the authors of the present invention observed that the substrates of the present invention promote an in-vivo-like morphology of the cells cultured on them with three-dimensional exposure of the cells, axons and synapses to the environment, while at the same time the neural network is accessible for functional and pharmacological screening. Moreover, by interfacing the substrate in cultured neuronal and glial cells, the formation of more complex organization and development of neuronal synaptic networks was observed.

### Method

An aspect of the present invention is directed to a method for promoting active neural cell network growth; preferably promoting active neural cell network 3D growth comprising the substrate of the present invention as described in any of its embodiments. In a particular embodiment said method is performed *in vitro* and/or *ex-vivo.* In another particular embodiment said method is performed *in vivo.*

### Culturing method

Another aspect of the invention is directed to an *in vitro* and/or *ex-vivo* culturing method comprising the steps:
i) providing
   a) the substrate of the present invention as described in any of its embodiments; and
   b) primary cultures developed from dissociated brain cells and organotypic cultures from explanted neural tissue; wherein the primary cultures comprise cells;
   wherein the substrate and the cells are in an *in vitro* and/or *ex-vivo* culture media; and
   wherein the textured surface with a plurality of protrusions of the substrate (a) is in contact with the surface of one or several cells (b) in the culture media;
ii) culturing the cells to obtain a neural cell network; and
iii) measuring the neural cell network activity.

In a particular embodiment, the culturing method of the present invention is performed in a confinement cavity comprising a culturing medium such as extracellular fluid medium.

In a particular embodiment, primary cultures of the present invention are developed from organotypic cultures of explanted neural tissue and/or from neuronal cells; preferably primary cultures developed from organotypic cultures of explanted neural tissue and neuronal cells.

In a particular embodiment, primary cultures of the present invention are developed from organotypic cultures of explanted neural tissue; more preferably from slice cultures of spinal cord and dorsal root ganglia (DRG); even more preferably from slice cultures of spinal cord.

In a particular embodiment, the cells of the present invention are neuronal cells of dissociated primary cultures.

In a particular embodiment, the cells of the present invention are neurons. The neurons used in the embodiment are not particularly limited as long as they can be cultured on the textured surface with a plurality of protrusions of the substrate of the present invention and may be appropriately selected from conventional neurons. For example, when they are applied to a human in the medical field, use may be made of neurons derived from a human progenitor cell, more specifically, neurons derived from the tissue to be transplanted to an affected portion of a recipient. On the other hand, when they are applied to a usage in which a human is not involved, for example, constructing a neuron system such as a neuro device or an artificial neural cell network, the neurons may not be derived from a human and may be selected from the neurons derived from various animal species and tissues; preferably rats. The neurons may be dissociated from a biological tissue or can be appropriately selected from available neurons suitable for the purpose.

In a particular embodiment, the cells of the present invention are primary cultures from neonatal rats hippocampi and/or organotypic cultures from spinal cord slices; more preferably are dissociated primary cultures.

In the context of the present invention, the expression "culture media" or "culture medium" is understood as known in the art, i.e. culture conditions suitable for the cells appropriately selected from known culture conditions.

In an embodiment, neural cell network or neurons can be cultured under culture conditions suitable for the neurons appropriately selected from known culture conditions. In a particular embodiment, neurobasal medium is used for culturing neurons. One skilled in the art would easily select culture conditions suitable for the selected neurons and perform culturing in the selected culture conditions.

In a particular embodiment, the step (ii) of culturing the cells to obtain a neural network comprises the following steps:
i. plating the cells of the present invention onto the substrate of the present invention; and
ii. incubating.

In a more particular embodiment, the step i comprises plating between 1 and 2000 cells/mm² onto the aforementioned substrates; preferably between 500 and 1000 cells/mm², more preferably around 800 cells/mm².

In a more particular embodiment, the step ii comprises incubating during between 1 h and 30 days; preferably between 1 and 15 days.

In a more particular embodiment, the substrate of the present invention was previously sterilized.

In an embodiment, the step (iii) of measuring the neural cell network activity comprises measuring the neural cell network electrical activity; preferably by imaging the calcium activity of the neural cell network; more preferably by using fluorescence calcium live imaging.

In a particular embodiment, measuring the neural cell network activity comprises measuring the neural cell network electrical activity; preferably the electrical activity that activates voltage-dependent ionic channels present in the membrane of the neural cells of the neural cell network leading to the occurrence of Ca²⁺ increase (such as an increase over the Ca²⁺ baseline) episodes, in particular by measuring the occurrence of spontaneous Ca²⁺ increase episodes; preferably by quantifying the peak-to-peak inter-event-interval (IEI) of the occurrence of spontaneous Ca²⁺ increase episode in seconds. By live calcium imaging, the activity of small groups of neurons is simultaneously monitored, detecting episodes of intracellular calcium rise, to measure the occurrence of neuronal and synaptic activity, abolished by tetrodotoxin (TTX) experiments. Neuronal calcium episodes are usually due to brief synchronous firing, leading to transient synchronization of synaptic events. These bursts are accepted measures of network dynamics in the art, even in the absence, in calcium imaging, of a single action potential or individual synaptic currents resolution.

In a more particular embodiment, the neural cell network activity comprises spontaneous Ca²⁺ increase episodes. In the context of the present invention, the spontaneous Ca²⁺ increase episodes of the neural cell network may be completely stopped by the addition of drugs, preferably by the addition of tetrodotoxin (TTX).

In the present invention, neural cell network activity and/or synchronization may be monitored by fluorescence calcium imaging said neural cell network; preferably representative regions. The occurrence of Ca²⁺ episodes or occurrences during the neural cell network activity and/or synchronization may be quantified by measuring the peak-to-peak inter-event-interval (IEI) in seconds; preferably during spontaneous activity. The IEI value distribution for different neural cell networks grown in different substrates may be compared. In addition, from said distribution (which is a statistically relevant distribution as known in the art) different parameters may be calculated such as the IEI median value and the IEI mean value. The calculated IEI values of the present invention may be shown in box-plots, wherein the thick horizontal bar indicates the median value, the cross indicates the mean value, and the boxed area extends from the 25th to 75th percentiles while whiskers from the 5th to the 95th percentiles. In a particular embodiment, the step (iii) of measuring the neural cell network activity comprises measuring the peak-to-peak inter-event-interval (IEI) median value of the neural cell network. In a more particular embodiment, the step (iii) further comprises comparing the IEI median value of the neural cell network with a certain IEI value. In a particular embodiment, if the IEI median value of the neural cell network is below a certain IEI value, then, the neural cell network is active; preferably, when the IEI median value is below 20s, more preferably below 10s; even more preferably is below 9s; even more preferably is around 8s.

Without being bound to a particular theory, the authors of the present invention have found that neural cell networks grown on the substrate of the present invention showed a different circuit-coupling efficacy (higher) than those growth on flat substrates, i.e. neural cell networks grown onto the substrates of the present invention exhibited enhanced spontaneous activity and synchronization, reminiscent of more complex 3D-neuronal networks, since 3D networks topology favors neuronal connectivity efficiency and transient synchronizations.

In a particular embodiment, measuring the neural cell network activity comprises measuring between non-neighboring cells of the neural cell network.

### Cultured neural cell network

Another aspect of the invention is directed to a cultured neural cell network obtainable by the culturing method described above in any of its particular embodiments; wherein the cultured neural cell network is active; preferably wherein the IEI median value as defined above is below 20s, more preferably below 10s; even more preferably, is around 8s.

In a particular embodiment, said neural cell network is active. In another particular embodiment, said neural cell network may by growth in vivo, in vitro or ex-vivo from explanted tissue; preferably in vitro or ex-vivo. Preferably, the neural cell network obtainable by the culturing method described above is an artificial neural cell network; more preferably is in vitro growth. Preferably, the neural cell network is not a culture of an in vivo developed brain or a tissue sample thereof.

In a particular embodiment, the cultured neural cell network obtainable by the method described above in any of its particular embodiments comprises between 2 and 1 million cells.

### Use

Another aspect of the invention is directed to the use of the substrate of the present invention comprising:
a) a textured surface with a plurality of protrusions, and
b) a bulk portion;
wherein both the textured surface and/or the bulk portion comprise a polymeric matrix;
wherein both the textured surface and/or the bulk portion comprise particles having a mean size of between 1 nm and 1000 µm,
wherein said particles are homogeneously distributed among the bulk portion and the textured surface; and
wherein the particles are electrically conductive,
for promoting neural cell network growth *in vitro* and/or *ex-vivo* and/or stimulation *in vitro* and/or *ex-vivo;* preferably for promoting neural network 3D growth *in vitro* and/or *ex-vivo* and/or stimulation of electrically excitable cells *in vitro* and/or *ex-vivo;* preferably electrical stimulation of electrically excitable cells *in vitro* and/or *ex-vivo.*

In the context of the present invention, the expression "electrically excitable cells" is understood as electrically active cell types that are able to generate an action potential in response to a depolarisation of the membrane potential above an excitation threshold.

In a particular embodiment, "electrically excitable cells" may be selected from neuronal, cardiac or muscle cells; preferably neural and/or cardiac cells; more preferably neural cells.

In a particular embodiment, electrically excitable cells are one or more neurons; preferably a neural cell network; more preferably an active neural cell network.

### Methods

In a particular embodiment, an aspect of the present invention is directed to a method for promoting neural cell network growth and/or stimulation
comprising the substrate of the present invention in any of its particular embodiments; wherein said substrate comprises:
a) a textured surface with a plurality of protrusions, and
b) a bulk portion;
wherein both the textured surface and/or the bulk portion comprise a polymeric matrix;
wherein both the textured surface and/or the bulk portion comprise particles having a mean size of between 1 nm and 1000 µm,
wherein said particles are homogeneously distributed among the bulk portion and the textured surface; and
wherein the particles are electrically conductive. In a particular embodiment, said method is performed *in vitro* and/or *ex-vivo.* In another particular embodiment said method is performed *in vivo.*

### Probe

An aspect of the present invention is directed to a probe comprising:
- the substrate of the present invention in any of its embodiments comprising:
   a) a textured surface with a plurality of protrusions, and
   b) a bulk portion;
   wherein both the textured surface and/or the bulk portion comprise a polymeric matrix; and
   wherein both the textured surface and/or the bulk portion comprise particles having a mean size of between 1 nm and 1000 µm, wherein said particles are homogeneously distributed among the bulk portion and the textured surface; and wherein the particles are electrically conductive; and
- an electrical connection comprising a conductive wire;
wherein the probe is optionally implantable.

In a particular embodiment, the probe is implantable.

In the context of the present invention, an "electrical connection" is understood as known in the art, for example, as a metal film or contact deposited by for example sputtering the metal onto any of the surfaces of the substrate of the present invention by means in the art. In addition, "a conductive wire" may be an electrically conductive wire such as a metallic wire as known in the art. Preferably, said electrical connection and/or wire may comprise metals as gold, silver, copper, platinum and/or combinations thereof. In a particular embodiment, the electrical connection of the probe is in the base of the bulk portion of the substrate.

In a particular embodiment, the substrate of the present invention is electrically conductive. In a more particular embodiment, the substrate of the present invention is able to pass current with applied voltage through either faradic or capacitive mechanisms and either through its textured surface or through its bulk portion. Preferably, the textured surface of the substrate of the present invention is in electrical contact with the bulk portion of the substrate. In a preferred embodiment, the textured surface of the substrate of the probe of the present invention is typically in electrical contact with the electrical connection of the probe.

The probe of the present invention may be used to stimulate, measure, or modulate cells; preferably for stimulation; more preferably for stimulation; even more preferably for electrical stimulation. The probe may be used as part of any other system, method or device that would benefit from stable, non-destructive access across a cell membrane. Similarly, the probes described herein may be used to form part of a biological interface for a medical device (such as an implant, prosthetic, or the like). In another particular embodiment the probe is implantable, preferably *in vivo.*

In an embodiment, the probe is an electrode. Thus, in a particular embodiment, the invention relates to an electrode with at least a surface comprising the substrate of the invention. Suitable electrodes that may comprise the substrate of the invention comprise those known in the field of electrical stimulation; preferably in the field of electrical neural stimulation.

In another particular embodiment, the probe is an electrode of an electrode pair as known in the art. In a more particular embodiment the electrode is the anode or the cathode; preferably the anode. For example, the probe of the present invention may be the anode having a positive potential with respect to the cathode. The polarity of the electrodes of the electrode pair may be changed (reversed relative to each other, or increased or decreased) during the course of an experiment, in particular during the course of electrical cell stimulation. The electrodes can therefore be used for electrical cell stimulation to promote electrical or electro-physiological activity (e.g. through changes in the cell membrane potential of electrically excitable cells). Such electrical conditions may occur under physiological conditions, *in vitro, ex-vivo* or in the presence of known or unknown drugs.

In another embodiment, the probe of the present invention is part of a medical device.

### Use of the probe

An aspect of the present invention is directed to the use of the probe of the present invention in any of its particular embodiments, for cell stimulation *in vitro* and/or *ex-vivo,* preferably for electrical cell stimulation, more preferably for electrical stimulation of electrically excitable cells; more preferably for electrical stimulation of electrically excitable cells in a cell culture media.

In a particular embodiment, when the probe of the present invention is in use as such or as part of another device in a cell culture media, the textured surface with a plurality of protrusions of the substrate is in contact with the surface of one or several cell; preferably in a culture media.

In the context of the invention, the probe of the present invention can be used for stimulation, preferably for "electrical stimulation"; preferably for electrical cell stimulation, in particular to cause depolarization on a cell, preferably on an electrically excitable cell.

In a preferred embodiment of the present invention, the probe is contacting the cell; preferably contacting the cell surface through the substrate of the present invention; more preferably through the textured surface of the substrate of the present invention. Electrical stimulation relies on the ability of "electrically excitable cells" to generate an action potential in response to a depolarisation of the membrane potential above an excitation threshold when a cell is in contact with, or impaled upon, a probe, for example, an electrode (e.g. during patch- or voltage- clamping), and an injection current or electrical pulse of a certain value (over a threshold) is applied. For example, a cardiomyocyte cell will require an injection current of about 1 nA to cause a membrane depolarisation of about 10 mV, and thereby generate an action potential. In similar circumstances, other electrically active cell types may require injection currents of between 1-10 nA to cause depolarisation. This in-situ electrical stimulation may allow the activation of voltage-dependent ionic channels present in the membrane of electrically excitable cells such as in neural cells or neurons. For example, it allows the control of key cellular messengers, such as calcium ions, that can lead to the stimulation of neural circuits in neurons. An expert in the art may determine the dosage and/or frequency of the injection current or electrical pulse of a certain value (over a threshold) applied which are effective according to the cells chosen.

In a particular embodiment, the probe of the present invention can be used for electrical stimulation; preferably for electrical cell stimulation, more preferably for electrical stimulation of electrically excitable cells, even more preferably *in vivo, ex-vivo* or *in vitro;* even more preferably *ex-vivo* or *in vitro.*

### Method

In a particular embodiment, the present invention is directed to a method for electrical stimulation of electrically excitable cells comprising the probe of the present invention in any of its particular embodiments. In a particular embodiment, said method is performed *in vitro* and/or *ex-vivo.* In another particular embodiment said method is performed *in vivo.*

### Stimulation device

An aspect of the present invention is directed to a stimulation device comprising
- the probe of the present invention as defined any of its embodiments; and
- an electrical pulse generator;
wherein the probe and the electrical pulse generator are electrically connected; and wherein the device is optionally implantable.

In the context of the invention, the term "implantable" is directed to a device that can be an insert or fix (tissue or an artificial object) in a person or animal, preferably by surgery. In another particular embodiment the stimulation device is implantable, preferably *in vivo.* In another more particular embodiment the stimulation device is part of a medical device.

In the context of the invention, the expression "electrical pulse generator" is directed to a device that is able to generate and deliver one or more unipolar pulses of varying strength, such as varying voltage (V), and duration (s) with varying frequency (Hz) to the probe of the present invention. The electrical pulse generator of the present invention may deliver one or more unipolar pulses or a voltage pulse train.

In a particular embodiment, the voltage pulse train delivered is between 1 and 50 s length and comprises pulses of between 1 and 10 ms duration being the pulses separated among them for a time interval of between 1 and 50 s; preferably, the voltage pulse train delivered is between 10 and 40 s length and comprises pulses of between 4 and 6 ms of duration and the pulses are separated among them for an interval of between 10 and 30 s.

In a more particular embodiment, the electrical pulses generated by the stimulation device of the present invention have intensities of between 0.01 and 100 V, preferably between 0.1 and 50 V, more preferably between 0.1 and 10 V.

The electrical pulse generator of the present invention may be a commercial pulse generator, for example a DigitimerTM pulse generator. The electrical pulse generator of the present invention may be triggered by a function generator. In addition, the current on the stimulating electrodes may be recorded. The threshold for excitation of the cell may be related to the geometry and surface of the probe as well as to the orientation of the cell in the electric field.

In another particular embodiment, the probe is an electrode of an electrode pair. In a more particular embodiment the probe is the anode or the cathode; preferably the anode. In a particular embodiment, the probe is the positive output of the stimulation device of the present invention

In a particular embodiment, the stimulation device of the present invention further comprises a ground output, preferably an electrode as a ground output, more preferably a Ag/AgCI pellet electrode as a ground output

### Use of the stimulation device

An aspect of the present invention is directed to the use of the stimulation device of the present invention in any of its particular embodiments for *in vitro* and/or *ex-vivo* cell stimulation; preferably for electrical cell stimulation and/or electrical cell neuromodulation *in vitro* and/or *ex-vivo;* more preferably for electrical neural cell stimulation and/or for electrical neural cell neuromodulation *in vitro* and/or *ex-vivo,* even more preferably for electrical cell growth stimulation and/or for electrical cell growth neuromodulation *in vitro* and/or *ex-vivo.*

In the context of the invention, the term "neuromodulation" is directed to the electrical modulation of the neural network activity for directing the electrical signaling and/or favoring the axonal regeneration, synapse formation and/or synapse plasticity.

In a particular embodiment, when the device of the present invention is in use as such or as part of another device in a cell culture media, the textured surface with a plurality of protrusions of the substrate of the probe of the present invention is in contact with the surface of one or several cell; preferably in a culture media.

### Method

In a particular embodiment, the present invention is directed to a method for electrical stimulation of electrically excitable cells comprising the stimulation device of the present invention in any of its particular embodiments. In a particular embodiment, said method is performed *in vitro* and/or *ex-vivo.* In a more particular embodiment, said method is performed *in vivo.*

### A monitor device

An aspect of the present invention is directed to a neural activity monitor device comprising
- the probe of the present invention as defined any of its embodiments or the stimulation device of the present invention as defined in any of the previous embodiments; and
- an electrical monitor;
wherein the probe and the electrical monitor are electrically connected.

In the context of the present invention, the expression "electrical monitor" is related to an electrical neural activity monitor.

In a particular embodiment, when the device of the present invention is in use as such or as part of another device in a cell culture media, the textured surface with a plurality of protrusions of the substrate of the probe of the present invention is in contact with the surface of one or several cells; preferably in a culture media.

In another particular embodiment the monitor device is implantable, preferably *in vivo.* In another more particular embodiment the stimulation device is part of a medical device.

In a particular embodiment, the electrical monitor of the present invention comprises a fluorescence detector; preferably a fluorescence imaging detector.

### Use of the monitor system

An aspect of the present invention is directed to the use of the monitor device of the present invention for cell monitoring *in vitro* and/or *ex-vivo;* preferably for electrical cell monitoring *in vitro* and/or *ex-vivo;* more preferably for electrical neural cell monitoring *in vitro* and/or *ex-vivo.*

In a particular embodiment, when the device of the present invention is in use as such or as part of another device in a cell culture media, the textured surface with a plurality of protrusions of the substrate of the probe of the present invention is in contact with the surface of one or several cell; preferably in a culture media.

### Method

In a particular embodiment, the present invention is directed to a method for electrical stimulation of electrically excitable cells comprising the monitor device of the present invention in any of its particular embodiments. In a particular embodiment, said method is performed *in vitro* and/or *ex-vivo.* In a particular embodiment, said method is performed *in vivo.*

### Compositions

### Composition 1

An aspect of the present invention is directed to a composition obtainable by the method comprising the steps of:
- providing
   ∘ the probe or the stimulation device or the monitor device of the present invention in any of its particular embodiments; and
   ∘ one or several electrically excitable cells in an *in vitro* and/or *ex-vivo* media;
- contacting the textured surface of the substrate of the probe with the surface of one or several electrically excitable cells; and
- electrically stimulating the one or several electrically excitable cells to obtain a composition;
preferably wherein said electrically excitable cells are preferably selected from neuronal, cardiac or muscle cells; preferably neural and/or cardiac cells; more preferably neural cells.

In a particular embodiment the contact and/or stimulation of the method to obtain the composition is performed *in vivo* or in an *in vitro* and/or *ex-vivo media;* particularly in an *in vitro* and/or *ex-vivo media.*

In a particular embodiment, the electrical stimulation is performed by applying one or more electrical pulses to the one or several electrically excitable cells.

### Composition 2

An aspect of the present invention is directed to a composition 2 comprising:
- the probe of the present invention in any of its particular embodiments or the stimulation device of the present invention or the monitor device of the present invention; and
- electrically excitable cells; preferably selected from neuronal, cardiac, or muscle cells; preferably neural and/or cardiac cells; more preferably neural cells;
wherein the textured surface of the substrate of the probe is in contact with the surface of one or several electrically excitable cells *in vivo* or in an *in vitro* and/or *ex-vivo media;* particularly in an *in vitro* and/or *ex-vivo media*

### Medical uses

In additional aspects, the present invention also refers to the composition 1 or 2 for use as a medicament or for use in medicine.

In particular, the present invention also refers to composition 1 or 2 for use in the treatment of chronic pain, epilepsy, and Parkinson's disease, sacral nerve pelvic disorders and incontinence, spinal cord traumatic disorders and spinal cord ischemic disorders (angina, peripheral vascular disease).

The present invention also refers to a method for treating chronic pain, epilepsy, and Parkinson's disease, sacral nerve pelvic disorders and incontinence, spinal cord traumatic disorders and spinal cord ischemic disorders (angina, peripheral vascular disease) in a subject in need thereof, the method comprising administering to the subject the composition 1 or 2 of the present invention.

The present invention also refers to the use of composition 1 or 2 of the present invention in the manufacture of a medicament for the treatment of chronic pain, epilepsy, and Parkinson's disease, sacral nerve pelvic disorders and incontinence, spinal cord traumatic disorders and spinal cord ischemic disorders (angina, peripheral vascular disease).

The present invention also refers to the use of composition 1 or 2 of the present invention in the treatment of chronic pain, epilepsy, and Parkinson's disease, sacral nerve pelvic disorders and incontinence, spinal cord traumatic disorders and spinal cord ischemic disorders (angina, peripheral vascular disease).

As used herein, the term "treatment" or derivations thereof include the eradication, removal, reversion, alleviation, modification, or control of a disease.

The subject or the subject in need which is treated is a human or animal subject, preferably a human subject.

In an embodiment, the invention is also directed to the prevention of a disease, and in particular chronic pain, epilepsy, and Parkinson's disease, sacral nerve pelvic disorders and incontinence, spinal cord traumatic disorders and spinal cord ischemic disorders (angina, peripheral vascular disease). The term "prevention" or derivations thereof refer to the avoiding or minimizing of the onset or development of the disease, in particular chronic pain, epilepsy, and Parkinson's disease, sacral nerve pelvic disorders and incontinence, spinal cord traumatic disorders and spinal cord ischemic disorders (angina, peripheral vascular disease).

The present invention is also directed to the medical use of composition 1 or 2 as described in any of the above embodiments, wherein the medical use is also as described in any of the above medical use embodiments.

### Method for treatment

The present invention is also directed to a method of treatment and/or prevention of a disease, which method comprises administering to a subject in need of such a treatment a therapeutically effective amount of electrical stimulation;
wherein said electrical stimulation is administered using the probe of the present invention in any of its embodiments, the stimulation device of the present invention in any of its embodiments, or the monitor device of the present invention in any of its particular embodiments.

In a particular embodiment of the present invention, the expression "administered" is related with the delivery of electrical stimulation; preferably of electrical pulses, in particular of an electrical pulse train.

The present invention is also directed to a method of treatment and/or prevention of chronic pain, epilepsy, and Parkinson's disease, sacral nerve pelvic disorders and incontinence, spinal cord traumatic disorders and spinal cord ischemic disorders (angina, peripheral vascular disease), which method comprises administering to a subject in need of such a treatment a therapeutically effective amount of electrical stimulation;
wherein said electrical stimulation is administered using the probe of the present invention in any of its embodiments, the stimulation device of the present invention in any of its embodiments, or the monitor device of the present invention in any of its particular embodiments.

In a particular embodiment, the electrical stimulation is electrical nerve stimulation.

In a particular embodiment, any of the methods of treatment and/or prevention of the present invention further comprises the following step:
i. applying a therapeutically effective amount of one or more electrical pulses, preferably to one or several electrically excitable cells of the subject in need; preferably to one or several neural cells of the subject in need.

In a particular embodiment, any of the methods of treatment and/or prevention of the present invention further comprises the following steps:
- positioning the substrate of the probe of the present invention in any of its embodiments, or the substrate of the stimulation device of the present invention in any of its embodiments, or the substrate of the monitor device of the present invention in any of its particular embodiments, into electrical contact with a selected portion of the subject in need of such a treatment, and
- applying a therapeutically effective amount of one or more electrical pulses.

In a particular embodiment, the subject in need of the present invention is a patient.

In a particular embodiment, any of the methods of treatment and/or prevention of the present invention are performed *in vivo.*

Non-limiting examples of portions of the subject in need of the treatment of the present invention are tissue of the subject comprising electrically excitable cells; preferably excitable cells selected from neuronal, cardiac, or muscle cells; preferably neural and/or cardiac cells; more preferably neural cells. For example, a portion of the subject in need might be selected from a portion of the brain, a portion of the spinal cord, nerves such as the sacral nerve, bundles of nerve fibers and nerve fibers; preferably a portion of the brain, the spinal cord or nerves

In a particular embodiment, the electrical stimulation of the method or treatment of the present invention is therapeutically effective.

In a particular embodiment, the electrical stimulation of the method or treatment of the present invention is for continuous administration.

In another particular embodiment, the electrical stimulation of the method or treatment of the present invention is for administration once every three months, once every two months, once a month, once every three weeks, once every two weeks, once a week, or once or several times a day.

### Medical device

The present invention is also directed to a medical device comprising the probe of the present invention in any of its embodiments, the stimulation device of the present invention in any of its embodiments, and/or the monitor device of the present invention in any of its particular embodiments; preferably an implantable medical device; more preferably a surgically implantable medical device.

In a particular embodiment the medical device of the present invention is implanted or catheter based; preferably permanently implanted.

In a more particular embodiment, the medical device of the present invention is implanted *in vivo.*

The present invention is also directed to a cardiac pacemaker, defibrillator, neurostimulator, cochlear implant, glucose monitor or implantable infusion pump comprising the probe of the present invention in any of its embodiments, the stimulation device of the present invention in any of its embodiments, or the monitor device of the present invention in any of its particular embodiments; preferably is implantable; more preferably is implantable *in vivo.*

In a particular embodiment, the present invention is directed to a neurostimulator comprising the probe of the present invention in any of its embodiments, the stimulation device of the present invention in any of its embodiments, or the monitor device of the present invention in any of its particular embodiments; preferably is implantable; more preferably is implantable *in vivo.*

### Kit

An aspect of the present invention is directed to a kit comprising:
- the probe of the present invention in any of its particular embodiments or the stimulation device of the present invention or the monitor device of the present invention;
- electrically excitable cells; preferably selected from neuronal, cardiac, or muscle cells; preferably neural and/or cardiac cells; more preferably neural cells; and
- a container; preferably a container comprising a suitable media for the cells;
wherein the textured surface of the substrate of the probe is in contact with the surface of one or several electrically excitable cells in an *in vitro* and/or *ex-vivo media.*

The following examples are merely illustrative of certain embodiments of the invention and cannot be considered as restricting it in any way.

An aspect (a) of the invention is directed to a method for promoting active neural cell network growth,
comprising a substrate comprising:
   a) a textured surface with a plurality of protrusions, and
   b) a bulk portion;
wherein both the textured surface and/or the bulk portion comprise a polymeric matrix.

In a particular embodiment, the aspect (a) of the present invention comprises the substrate of the present invention in any of its particular embodiments. In another particular embodiment, the aspect (a) is performed *in vivo,* or in an *in vitro* and/or *ex-vivo* media; particularly in an *in vitro* and/or *ex-vivo* media.

Another aspect (b) of the present invention is directed to a culturing method comprising the steps:
i) providing
   a) the substrate as defined in claim 1; and
   b) primary cultures developed from dissociated brain cells and organotypic cultures from explanted neural tissue; wherein the primary cultures comprise cells;
   wherein the substrate and the cells are in a culture media; and wherein the textured surface with a plurality of protrusions of the substrate (a) is in contact with the surface of one or several cells (b) in the culture media;
ii) culturing the cells to obtain a neural cell network; and
iii) measuring the neural cell network activity .

In another particular embodiment, the aspect (b) is performed *in vivo,* or in an *in vitro* and/or *ex-vivo* media; particularly in an *in vitro* and/or *ex-vivo* media.

Another aspect (c) of the present invention is directed to a cultured neural cell network obtainable by the culturing method according to aspect (b); wherein the artificial neural cell network is active.

Another aspect (d) of the present invention is directed to a method for promoting neural cell network growth and/or stimulation
comprising the substrate of the present invention in any of its particular embodiments; wherein said substrate comprises:
   a) a textured surface with a plurality of protrusions, and
   b) a bulk portion;
wherein both the textured surface and/or the bulk portion comprise a polymeric matrix;
wherein both the textured surface and/or the bulk portion comprise particles having a mean size of between 1 nm and 1000 µm,
wherein said particles are homogeneously distributed among the bulk portion and the textured surface; and
wherein the particles are electrically conductive.

In another particular embodiment, the aspect (d) is performed *in vivo,* or in an *in vitro* and/or *ex-vivo* media; particularly in an *in vitro* and/or *ex-vivo* media.

An aspect (e) of the present invention is directed to a probe comprising:
- the substrate of the present invention in any of its embodiments comprising:
   a) a textured surface with a plurality of protrusions, and
   b) a bulk portion;
   wherein both the textured surface and/or the bulk portion comprise a polymeric matrix; and
   wherein both the textured surface and/or the bulk portion comprise particles having a mean size of between 1 nm and 1000 µm, wherein said particles are homogeneously distributed among the bulk portion and the textured surface; and wherein the particles are electrically conductive; and
- an electrical connection comprising a conductive wire;
wherein the probe is optionally implantable; preferably *in vivo.*

Another aspect (f) of the present invention is directed to a method for electrical stimulation of electrically excitable cells comprising the probe of the present invention in any of its particular embodiments. In another particular embodiment, the aspect (f) is performed *in vivo,* or in an *in vitro* and/or *ex-vivo* media; particularly in an *in vitro* and/or *ex-vivo* media.

An aspect (g) of the present invention is directed to a stimulation device comprising
- the probe of the present invention as defined any of its embodiments; and
- an electrical pulse generator;
wherein the probe and the electrical pulse generator are electrically connected; and wherein the device is optionally implantable; preferably *in vivo.*

An aspect (h) of the present invention is directed to a method for electrical stimulation of electrically excitable cells comprising the stimulation device of the present invention in any of its particular embodiments. In another particular embodiment, the aspect (h) is performed *in vivo,* or in an *in vitro* and/or *ex-vivo* media; particularly in an *in vitro* and/or *ex-vivo* media.

An aspect (i) of the present invention is directed to a neural activity monitor device comprising
- the probe of the present invention as defined any of its embodiments or the stimulation device of the present invention as defined in any of the previous embodiments; and
- an electrical monitor;
wherein the probe and the electrical monitor are electrically connected; wherein the device is optionally implantable; preferably *in vivo.*

Another aspect (k) of the present invention is directed to a method for electrical stimulation of electrically excitable cells comprising the monitor device of the present invention in any of its particular embodiments. In another particular embodiment, the aspect (k) is performed *in vivo,* or in an *in vitro* and/or *ex-vivo* media; particularly in an *in vitro* and/or *ex-vivo* media.

Another aspect (I) of the present invention is also directed to a method of treatment and/or prevention of a disease, which method comprises administering to a subject in need of such a treatment a therapeutically effective amount of electrical stimulation;
wherein said electrical stimulation is administered using the probe of the present invention in any of its embodiments, the stimulation device of the present invention in any of its embodiments, or the monitor device of the present invention in any of its particular embodiments.

In another particular embodiment, the aspect (I) is performed *in vivo.*

Another aspect (m) of the present invention is directed to a method of treatment and/or prevention of chronic pain, epilepsy, and Parkinson's disease, sacral nerve pelvic disorders and incontinence, spinal cord traumatic disorders and spinal cord ischemic disorders (angina, peripheral vascular disease), which method comprises administering to a subject in need of such a treatment a therapeutically effective amount of electrical stimulation;
wherein said electrical stimulation is administered using the probe of the present invention in any of its embodiments, the stimulation device of the present invention in any of its embodiments, or the monitor device of the present invention in any of its particular embodiments.

In another particular embodiment, the aspect (m) is performed *in vivo.*

Another aspect (n) of the present invention is directed to a medical device comprising the probe of the present invention in any of its embodiments, the stimulation device of the present invention in any of its embodiments, or the monitor device of the present invention in any of its particular embodiments; wherein the device is optionally implantable; preferably *in vivo.*

Another aspect (o) of the present invention is directed to a neurostimulator device comprising the probe of the present invention in any of its embodiments, the stimulation device of the present invention in any of its embodiments, or the monitor device of the present invention in any of its particular embodiments; wherein the device is optionally implantable; preferably *in vivo.*

### EXAMPLES

The invention is illustrated by means of the following examples that in no case limit the scope of the invention.

### Example 1 - Preparation of polymer substrates comprising nanotextures by thermal nanoimprint lithography (NIL)

The nanostructured substrates were prepared by thermal nanoimprinting using polydimethylsiloxane (PDMS) working molds. These were obtained by soft lithographic replication of a silicon master mold produced by lithographic and deep reactive ion etching techniques. The thermal imprinting conditions for the textured substrates were: T = 140 °C; P = 30 bar and t = 5 min. Figure 2a shows an example of the substrates fabricated in the present example.

### Example 2 - Preparation of polymer nanocomposite substrates comprising carbon-based particles by different methods and thermal nanoimprint lithography (NIL)

Nanocomposite substrates comprising carbon-based particles were prepared using three synthesis methods: a) in-situ radical polymerization; b) solution mixing; and c) melt mixing; followed by nanoimprinting (Figure 1), as follows.

### Example 2a -In-situ radical polymerization

A polystyrene nanocomposite film comprising a 0.35 wt% of single wall carbon nanotubes (SWCNT) [synthesized by the HiPco method, CNI Technology. TX, USA] embedded in said polymer, was obtained as follows. Styrene was processed by in situ radical polymerization using azobisisobutyronitrile as initiator. After oxygen was removed by freeze-thawing cycles, a reaction flask containing styrene and SWCNT was placed into an oil bath preheated at 60°C. The polymerization reaction of styrene was performed under inert atmosphere during 20 hours with constant stirring. After that, a composite comprising SWCNT and polystyrene was coagulated by drop wise addition of the solutions into a large volume of methanol (MeOH) under intense stirring. The composite precipitated instantly due to the poor polystyrene solubility in MeOH while the nanotubes were entrapped by the polymer chains in the process. The composite was then filtered, washed repeatedly with MeOH and dried in vacuum overnight. Then, composite films (ca. 200 µm thickness) were made by a melt pressing method using a heating stage. Then, nanostructured substrates were prepared by thermal nanoimprinting using PDMS working molds (obtained from a Silicon master mold as described in Example 1). The thermal imprinting conditions for the nanosubstrates were: T = 140 °C; P = 30 bar and t = 5 min.

The nanostructured substrate obtained comprised pillar arrays with high aspect ratio (lengths about 2 microns and diameters about 500 nm) with distances among them of about 500 nm and a bulk flat portion. The surface and the bulk portion of the substrate comprised a polymeric matrix with 0.35 wt% of SWCNT and were conductive.

### Example 2b -Solution mixing.

A polystyrene nanocomposite film comprising a 1 wt% of single wall carbon nanotubes (SWCNT) [P3-SWNT product type, Carbon Solutions, Inc], was obtained by a solvent mixing method as follows. SWCNTs were dispersed in toluene or dimethyl formamide (DMF) and then mixed with a polymer solution prepared using the same solvents. Several sonication and dispersion cycles were performed to enhance the SWCNTs dispersion (sonication tip and Ultraturrax tools). Subsequently, a nanocomposite of polystyrene comprising SWCNT was coagulated in MeOH and dried at 80°C under vacuum for 24h. Nanocomposite films (ca. 200 µm thickness) were made by a melt pressing method using a hot-press.

Then, nanostructured nanocomposite substrates were prepared by thermal nanoimprinting using the PDMS working molds (described in example 1). The thermal imprinting conditions were: T = 140 °C; P = 30 bar and t = 5 min.

The nanostructured substrates obtained comprised pillar arrays with high aspect ratio (lengths about 2 microns and diameters about 500 nm) with distances among them of about 500 nm and a bulk flat portion. The surface and the bulk portion of the substrate comprised a polystyrene matrix with 1 wt% of SWCNT and were conductive.

### Example 2c - Melt mixing.

A polystyrene nanocomposite film comprising a 1-3 wt% of single wall carbon nanotubes (SWCNT) [P3-SWNT product type, Carbon Solutions, Inc], was obtained by a melt mixing method as follows. The SWCNT were mixed with the matrix and feed into a compounder or extruder. The mixing was performed at temperatures well above the Tg (glass transition temperature) or Tm (melting temperature) of the polymer matrix. The shear forces generated during the extruding process allow breaking the SWCNT bundles to obtain a good dispersion of them in the polymeric matrix. Composites with high SWCNT content (up to 3% w/w) were obtained using a micro-compounder twinscrew extruder and mixing for 20 minutes prior extrusion at T=140-160°C and 100 rpm. Nanocomposite films (ca. 200 µm thickness) were made by a melt pressing method using a heating stage.

Then, nanostructured nanocomposite substrates were prepared by thermal nanoimprinting using PDMS working molds (as described in example 1). The thermal imprinting conditions were: T = 140 °C; P = 30 bar and t = 5 min.

The nanostructured substrates obtained comprised pillar arrays with high aspect ratio (lengths about 2 microns and diameters about 500 nm) with distances among them of about 500 nm and a bulk flat portion. The surface and the bulk portion of the substrate comprised a polystyrene matrix with 1 wt% of SWCNT and were conductive. Figure 2b shows an example of the substrates fabricated in the present example. The presence of carbon-based particles (carbon nanotubes) within the nanostructured substrates was confirmed by scanning electron microscopy (SEM) and RAMAN spectroscopy. The signal of the carbon based particles inside the nanopillars was detected by confocal RAMAN spectroscopy with a depth resolution of around 400 nm.

### Example 3 - Characterization of dissociated primary cultures from neonatal rats hippocampi and organotypic cultures from spinal cord slices grown on different substrates.

Dissociated cells obtained from P2-P3-old Wistar rats hippocampi were cultured and grown on substrates with different textures (flat or nanostructured) and compositions (polystyrene or a composite of a polystyrene matrix comprising SWCNTs) as follows.

Dissociated cells were obtained from P2-P3-old Wistar rats hippocampi by subsequent steps of enzymatic and mechanic digestion.

Three different substrates were used: a flat polystyrene film (PS Flat), a nanostructured substrate of polystyrene (PS) and a nanostructured substrate of polystyrene-SWCNTs (PS-CNTs) of the example 2a. The nanostructured substrates comprised pillar arrays with high aspect ratio (lengths about 2 microns and diameters about 500 nm) with distances among them of about 500 nm and a bulk portion.

The substrates were sterilized with three 5 minutes-long washes on milliQ H₂O previously filtered with 0.22 µm cutoff filter (Merck Millipore) and dried at 60 °C. Their surfaces were activated under low-pressure air plasma (Harrick PDC-32G Plasma Cleaner) for 7 minutes at RT, with RF coil powers of 9 Watt. A 20 minutes-long exposition to ultraviolet (UV)-radiation was finally used to sterilize the substrates.

About 800 cells/mm² were plated onto the aforementioned substrates and incubated at 37°C, 5% CO₂ in Neurobasal medium (Invitrogen) added with B-27 supplement (Thermo Fisher) and GlutaMAX (Thermo Fisher) both to a 1X final concentration. Gentamicin (Thermo Fisher) was also added to a final concentration of 5 µg/ml to prevent contaminations.

Cultured cells grown for 8-12 days in vitro (DIV) were subsequently studied by scanning electron microscopy (SEM), immunocytochemistry, fluorescent microscopy and calcium imaging as follows.

*Sample characterization and results by scanning electron microscopy (SEM): interaction between neurons and different substrates.*

SEM micrographs were acquired collecting secondary electrons on a Gemini SUPRA 40 SEM (Carl Zeiss NTS GmbH, Oberkochen, Germany). Primary cultures onto PS and PS-CNTs substrates were fixed in with 2% glutaraldehyde dissolved in cacodylate buffer (0.1 M, pH 7.2) in the dark for 1 hour at room temperature. After fixation samples were carefully rinsed with cacodylate buffer; dehydration process followed by washing the samples in water/ethanol solutions at progressively higher alcohol concentrations (25%, 50%, 75%, 95% and 100% ethanol for 3 minutes each). Samples were dried at room temperature in a N₂ chamber overnight. Prior to SEM imaging samples were metalized in a metal sputter coater (Polaron SC7620). In order to prevent electron-induced surface charging, low accelerating voltages (2 keV) were used for cells visualization.

Results showed that, after 8 to 12 days in vitro (DIV), neonatal rat hippocampal cells successfully adhered and matured in flat and nanostructured substrate of PS and in PS-CNTs nanostructured substrate (Figure 3a and Figure 4a).

Scanning electron microscopy (SEM) was used to assess the physical interaction of neuronal membranes with the underlying nanopillar substrate (PS and PS-CNTs). A prominent process (axons and dendrites) branching was evident at relatively low magnification as shown in Figure 3a (left). Neurons ability to tilt single nanopillars (Figure 3a, middle) was interpreted as a "proof of strength" for cytoskeletal components, linked to neuronal healthiness and regenerative capability. The nanostructured substrates (PS and PS-CNTs) allowed peculiar network architectures where neuronal cell bodies and processes were visibly suspended (Figure 3a, right), facing a pseudo-3D extracellular environment.

### Samples characterization and results by immunocytochemistry an fluorescent microcopy

Cultures fixed in 4% paraformaldehyde in PBS 1X were permeabilized for 30 min with 0.3% Triton-X-100 (Carlo Erba) in PBS 1X added with 5% FBS (Gibco) and 4% BSA (Sigma-Aldrich). Samples were mixed with primary antibodies for 30 min at room temperature (RT) and being washed with PBS 1X, with secondary antibodies for 45 minutes. Mounting was performed with anti-fade medium Fluoromount (Sigma-Aldrich) onto microscope glass slides. Neurons were labeled with anti-β-tubulin III primary antibody (1:600, Sigma-Aldrich) and visualized with Goat Anti-Rabbit Alexa 594 as secondary antibody (1:800, Invitrogen). Astrocytes were stained with mouse anti-GFAP primary antibodies (1:600, Sigma-Aldrich) and visualized with Goat anti-Mouse Alexa 488 as secondary antibody (1:800, Invitrogen). Nuclei were stained with 4',6-diamidino-2-phenylindole (DAPI, 1:800, Invitrogen). Images of immunolabeled cultures were acquired using a Leica DM6000 Epifluorescence Microscope either with a 10x or with a 20x objective (HC PL Fluotar, with 0.30 and 0.50 NA respectively). Cells and nuclei count were performed manually on collected fields (at least 8 field per culture). Analysis and images reconstruction were accomplished using NIS-Elements (Nikon) and ImageJ (NIH) software.

Neuronal and glial phenotypes were visualized by immunofluorescence imaging of a specific cytoskeletal component: β-tubuiin III to visualize neurons, and glial fibrillary acidic protein (GFAP) to visualize astrocytes (Figure 3b, red and green labels respectively). PS and PS-CNTs nanostructured substrates showed a comparable density of β-tubuiin III positive neurons (253 ± 115 and 264 ± 59 cells/mm², respectively) compared to control flat surfaces (258 ± 78 cells/ mm²; Figure 3c, right plot) indicating a good biocompatibility of both the polymer and the nanostructured substrate. In the same set of cultures total DAPI-stained nuclei (Figure 3b, blue label) were counted and β-tubuiin III positive ones were subtracted to obtain an estimate of neuroglial nuclei. A marked reduction of these latter was found in both PS and PS-CNTs (774 ± 347 and 675 ± 227 neuroglial nuclei/mm2, respectively, ***P < 0.001) compared to control glass (1113 ± 249 neuroglial nuclei/mm2; Figure 3c, right).

β-tubuiin III-positive and GFAP-positive cells density were also studied in another subset of cultures in two substrates: poly-L-ornithine-coated glass coverslips (control) and flat polystyrene surfaces (PS-flat), Figure 4a, left panels.

Figure 4 (a-b) shows the neuronal and astrocytes densities in control poly-L-ornithine coated flat glass (control) and flat polystyrene surfaces (PS-flat). (a) From left to right, a representative immunostaining (β-tubuiin III in red, GFAP in green, DAPI in blue; scale bar = 50 µm), a snapshot of Oregon green-loaded cultures (scale bar = 100 µm) and the corresponding Ca²⁺ imaging traces of spontaneous activity prior and after adding tetrodotoxin. (b) Box-plots of neuronal and astroglial densities expressed as number of cells/mm².

Cell population densities were similar on PS flat surfaces (205 ± 58 neurons/mm² and astrocytes-to-neurons ratio of 1.36 ± 0.3) compared to control (229 ± 55 neurons/mm², and astrocytes-to-neurons ratio of 1.34 ± 0.23, Figure4b).

In addition, when studying the network activity, PS-flat did not induce changes in neural cell network activity when compared to control flat glass (see Figure 4A).

### Calcium imaging samples characterization

Hippocampal dissociated cultures were loaded with cell permeable Ca²⁺ dye Oregon Green 488 BAPTA-1 AM (Molecular Probes) at a final concentration of 4 µM for 30 min at 37 °C, 5% CO2. Samples were mounted on an inverted microscope (Nikon Eclipse Ti-U) where cultures were continuously perfused at 5 mL/min rate and at RT with extracellular saline solution of composition (mM): 150 NaCl, 4 KCI, 2 CaCI2, 1 MgCI2, 10 HEPES, 10 glucose (pH adjusted to 7.4 with NaOH; osmolarity 300 mOsm). Ca²⁺-dye was excited at 488 nm with a mercury lamp; excitation light was separated from the light emitted from the sample using a 505 nm dichroic mirror and ND filter (1/32). Cultures were observed with a 20X objective (PlanFluor, 0.45 NA) and images were acquired with exposure time of 150 ms using an ORCA-Flash4.0 V2 sCMOS camera (Hamamatsu). The imaging system was controlled by an integrating imaging software (HClmage Live) and the camera was set to operate on 2048x2048 pixels at binning 4. Cultures accustomed to extracellular solution for about 10 minutes. Spontaneous activity was therefore recorded for 10 minutes. 10 µM bicuculline (GABA_{A} antagonist; Sigma-Aldrich) was subsequently perfused for 20 minutes to weaken the synaptic inhibition and increase the synchronization. Finally, 1 µM TTX (a voltage-gated, fast Na+ channel blocker; Latoxan) was added to confirm the neuronal nature of the recorded signals. 1 field was recorded from each sample and 10 ± 2 cells from each recording were selected by drawing regions of interest (ROIs) around cell bodies. Images were analyzed with ImageJ software (NIH) and the corresponding traces were studied with Clampfit software (pClamp suite, 10.4 version; Axon Instruments) in off-line mode. The difference between consecutive peaks onset times was computed, to obtain the inter-event interval (IEI). Intracellular Ca²⁺ transients were expressed as fractional amplitude increase, where F0 is the baseline fluorescence level and ΔF is the rise over baseline.

The synchronization between non-neighboring cells in the same field from a calcium imaging experiment was determined with a MATLAB (MathWorks, Inc.) custom program were the traces of calcium activity were analyzed in all pairs of cells. The rustling pairs of traces were divided in 20 chunks, reiterating 10 000 times the process after shifting randomly the starting time window. For all the generated the Pearson Correlation Coefficient (CCF) was estimated, generating a distribution of CCFs with its mean (real CCFs mean). All the previously generated chunks were shuffled among them and a distribution of randomly generated CCFs was obtained. The real CCFs mean was therefore plotted over the distribution of randomly generated CCFs and the extent of the area of the random CCF that exceeds the real CCFs mean allows the calculation of a p-value. When the significance was under the 0.05 threshold signals were considered synchronized. Significantly correlated pairs were count and used to estimate the percentage of total cells pair synchronized, Figure 5 (b) bottom bar plot indicate the augmented percentage of synchronized neurons in PS and PS_CNT when compared to control.

The formation of active synaptic neuron networks was studied by simultaneously imaging the intracellular calcium activity of living neurons (8-12 DIV) grown on different substrates: flat poly-L-ornithine-coated glass coverslips (control); nanostructured substrates of polystyrene (PS) and nanostructured substrates of polystyrene-CNTs (PS-CNTs).

To monitor neuronal activity with fluorescence calcium imaging, representative regions of 660 x 660 µm were sampled. Neurons stained with the membrane permeable Ca²⁺ dye were visualized within the sampled area and 10 ± 2 fluorescent cells were selected as ROIs (region of interest).

The neurons network grown in the three different substrates were synaptically connected and displayed spontaneous activity including bursts emerging by irregular synchronized firing epochs. The spontaneous bursting activity was fully blocked by adding Tetrodotoxin (1 µM) (see Figure 4a, 5a, 7a,a and Figure 8), confirming the neuronal nature of all recorded signals.

The occurrence of spontaneous Ca²⁺ episodes in active neuronal cells was measured by quantifying the peak-to-peak inter-event-interval (IEI) in seconds. IEIs distribution was different in neuronal cultures grown on nanostructured substrates (both PS and PS-CNT substrates) when compared to those grown onto glass coverslips (***P < 0.001; upper box plot in Figure 5b) suggesting a different circuit-coupling efficacy. Median IEI values were 8.8 s and 8.1 s for PS and PS-CNTs, respectively, and 12 s for control poly-L-ornithine-coated glass coverslips (upper box plot in Figure 5b). Thus, neuronal cultures grown onto nanostructured substrates exhibited enhanced spontaneous activity in a substrate topography dependent-manner.

Significantly correlated pairs of cells (bottom bar plot in Figure 5b) expressed as percentage of total cells pair, were analyzed. Calcium events correlation increased significantly from a 46% in control glass cultures to 86% and 96% in neuronal cultures grown onto nanostructured substrates (PS and PS-CNTs respectively, Figure 5b, bottom).

### Example 4 - Electrical stimulation using nanostructured substrates

A PS-CNTs nanostructured substrate of the example 2b was used as the interface of a stimulating electrode for neuronal extracellular stimulation. Dissociated hippocampal cultures were grown for 10 DIV in the same conditions described above (see Example 3). Cells were loaded with the Ca²⁺ dye described above, and after incubation, the petri dish with the substrate was mounted in a fixed-stage upright microscope (Eclipse FN1, Nikon) and was used as perfusion chamber during the experiment (see Figure 6). Spontaneous calcium transients were recorded with a 20 x water immersion objective (UMPlanFI, 0.5 NA, Olympus) using an EMCCD camera (IXon Ultra 897, AndorTM, Oxford Instruments) controlled by a computer through NIS-elements D (Nikon). Images were acquired each 150 ms at 10 MHz readout compensating the read noise with x250 EM gain.

After about 15 minutes of spontaneous activity, synaptic blockers (all from Sigma-Aldrich) APV 25 µM (NMDA receptor selective antagonist), bicuculline 10 µM (GABA_{A} receptor antagonist) and CNQX 10 µM (AMPA/kainate receptor antagonist) are applied completely silencing the cell neural network spontaneous activity. In these conditions only evoked signals were visible in the Ca²⁺ imaging experiment.

An isolated electrical stimulator (Digitimer DS2A) was used for the electrical cell stimulation. The positive output of the stimulator was plugged to a stimulating platinum wire in contact with the PS-CNTs substrate from its bottom side, while the ground output was plugged with an Ag/AgCI pellet electrode submerged in an extracellular saline solution (Figure 6). A voltage pulse train was generated with the electrical stimulator. ImageJ software (NIH) and Clampfit software (pClamp suite, 10.4 version; Axon Instruments) in off-line mode were used to analyze the data recorded.

Electrical Stimulation from the conductive PS-CNTs nanostructured substrate acting as an interface between the cells and the stimulator was achieved (Figure 7). After baseline spontaneous activity recording (representative trace, Figure 7a), the presence of a specific cocktail of synaptic blockers, resulted in a silenced network where any electrically evoked activity was efficiently measured from the cultured neurons (a smaple cell is shown in Figure 7c tracings). Figure 7b shows a 25 second-long pulse train, which when applied to the cell culture, resulted in strong and transient increases in the Ca²⁺ dye fluorescence (Figure 7c) caused by the applied electric field. Such responses were eliminated in the presence of tetrodotoxin, thus confirming the neuronal nature of the signals and indicating that such rise in Ca²⁺ was triggered by evoked action potentials.

### Example 5 - Organotypic slice cultures Spinal Cord

Spinal cord slices were obtained from embryos at 12-13 days of gestation (E12-13) from a pregnant female mouse (C57BI/6). The thoracolumbar portion of the spinal cord was explanted and cut into transversal slices (275 µm) with a tissue chopper (Mcllwain TC752, Campden instruments Ltd.). Slices were plated either onto glass coverslips or the tested materials (PS and PS-CNTs) by clotting 15 µL of chicken plasma with 23 µL of thrombin (both from Sigma-Aldrich). Organotypic Spinal Cords were grown with 1 mL medium containing 66% DMEM 1X (Gibco), 8% sterile water for tissue culture, 25% fetal bovine serum (Gibco), 1% antibiotic-antimycotic (Gibco), 2% B-27 supplement (Gibco) and 20 ng/mL nerve growth factor (NGF, Alomone Laboratories); 300 mOsm; pH 7.35. Cultures were kept in a roller drum at 120 revolution per hour in an incubator at 37°C and 5% CO2 for about 12-21 days in vitro (DIV). After 7 DIV culture medium was replaced for 24h by fresh medium containing antimitotics (10 µM ARA-C/Uridine/5-FI-dU) and reduced NGF concentration (5 ng/mL). At DIV 8 fresh medium with reduced NGF was added and refreshed every 7 days.

Organotypic slices were fixed in 4% paraformaldehyde in PBS 1X for at least 1h. After fixation slices were incubated for 10 min with 0.1 M glycine in PBS 1X and permeabilized for 1h with 0.3% Triton-X-100 (Carlo Erba) in PBS 1X added with 5% FBS (Gibco) and 4% BSA (Sigma-Aldrich) to prevent non-specific binding of primary antibodies. Samples were subsequently incubated with primary antibodies overnight at 4 °C and with the secondary antibodies for 2h at RT. Mounting was performed with anti-fade medium Fluoromount (Sigma-Aldrich) on microscope glass slides. All neurons were labeled through anti-β-tubulin III primary antibody (1:600, Sigma-Aldrich) and visualized with Alexa 594 anti-rabbit in goat as secondary antibody (1:800, Invitrogen). Exclusive staining of motoneurons was instead achieved with anti-neurofilament H (SMI-32, 1:600, Biolegend) recognized by Alexa 488 anti-mouse in goat as secondary antibody (1:800, Invitrogen). Nuclei were stained with 4',6-diamidino-2-phenylindole (DAPI, 1:800, Invitrogen). A Nikon Eclipse Ti2 inverted microscope connected to a A1R confocal system (Nikon Instruments) was used to acquire stitched images at 4x, 10X and 20X magnification (Plan Apo λ, with 0.20, 0.45 and 0.75 NA respectively) to obtain a morphological insight of the stained spinal cord slice cultures.

Organotypic spinal cord, grown in vitro for 3 weeks, were loaded with cell permeable Ca²⁺ dye Fluo-4 AM (Molecular Probes); 11.6 µL of DMSO (Sigma-Aldrich) were added to the stock 50 µg of the dye and cultures were incubated with a final concentration of 4 µM for 1h in the roller drum at 37 °C, 5% CO2. After dye loading a de-esterification period followed, cultures were maintained in extracellular saline solution (described above) in the same incubator for 30 min. Samples were mounted on the previously described inverted microscope (Nikon Eclipse Ti-U). The dye was excited at 488 nm and the emission was detected at 520 nm. Cultures were continuously perfused with extracellular saline solution and neurons at the premotor region in the ventral zone of the slice were observed with a 40X objective (PlanFluor, 0.60 NA). Images were acquired every 150 ms using the ORCA-Flash4.0 V2 sCMOS camera (Hamamatsu) operating at binning 4. After cultures accustomed to extracellular solution, spontaneous activity was recorded for 10 minutes and afterwards 1 µM TTX (a voltage-gated, fast Na+ channel blocker; Latoxan) was added to confirm the neuronal nature of the recorded signals. Images were analyzed with ImageJ software (NIH) and the corresponding traces were extracted with Clampfit software (pClamp suite, 10.4 version; Axon Instruments) in off-line.

The long-term biocompatibility of polymeric nanostructured substrates was tested by culturing organotypic spinal cord slices obtained from mouse embryos at days 12-13 of gestation using immunofluorescence labeling and confocal microscopy (Figure 8, left panels) to investigate the adhesion, development and morphology of spinal explanted tissue and axonal re-growth, when chronically interfaced to the substrates for 3 weeks. Spinal explants grown onto PS and PS-CNTs nanostructured substrates were compared to control ones, grown on standard growth supports (flat glass coverslips). The neuron-specific cytoskeletal components microtubules (by anti-β-tubulin III; in red) and the axonal neurofilament H (by Smi-32, in green). Nuclei are visualized by DAPI (in blue; scale bar 500 µm) were imaged by immunofluorescence.

Results showed that adhesion, growth and morphology of explanted spinal cord cultures were not altered by the nanopillars of the nanostructured substrates. Thus, by this long-term assessment, the ability of such substrates to support spinal cord adhesion and axonal re-growth was demonstrated.

The impact in spinal slices functioning wherein in contact with nanostructured PS and PS-CNT substrates was tested. Spinal organotypic slices displayed, after 3 weeks of culturing, an intense spontaneous synaptic activity. Said spontaneours activity was monitored via live calcium fluorescence imaging. In particular, as spontaneous bursts of irregular synchronized firing epochs visualized as spontaneous fluctuations of intracellular calcium levels. Spinal-cord cultures were loaded with Ca²⁺ Fluo-4 AM dye as described above and the network activity was monitored by fluorescence calcium imaging. As depicted in the right images of Figure 8 (scale bar 50 µm), fluorescence cells were detected within the ventral area (pre-motor area) of each slice culture. Then, spontaneous neuronal activity was measured by imaging the intracellular calcium fluorescence dynamic of selected neurons (Figure 8, middle tracings). Said activity was fully blocked by Tetrodotoxin (1 µM; Figure 8, right tracings).

The peak-to-peak inter-event-interval (IEI) occurrence of spontaneous Ca²⁺ episodes in active cells was quantified (in seconds). It was observed that the IEI data distribution was about 3-fold smaller in organotypic spinal cord cultures on nanostructured PS and PS-CNT substrates than those grown onto glass coverslips (control). This confirms an improvement in the coupling efficiency of the spinal premotor circuit, even after 3 weeks of contact between the cultures and the nanostructured substrates. Thus, this experiment probes the ability of nanostructured substrates to act as stable long-term spinal cord interfaces.

### Example 6 - Statistical analysis

All values from samples subjected to the same experimental protocols in previous experiments were pooled together and results are presented as mean ± S.D., if not stated otherwise, with n = number of neurons. A statistically significant difference between two data sets was assessed by Student's t test (after checking variances homogeneity by Levene's test) for parametric data and by Mann-Whitney's test for non-parametric ones. Two-way analysis of variance (two-way ANOVA) and one-way ANOVA were used for parametric data or Kruskal-Wallis test for non parametric ones, to determine significance when multiple groups were compared. Statistical significance was determined at P < 0.05.

In box-plots, the thick horizontal bar indicates the median value, the cross indicates the mean value, the boxed area extends from the 25th to 75th percentiles while whiskers from the 5th to the 95th percentiles.

## Claims

1. Use of a substrate comprising:
a) a textured surface with a plurality of protrusions, and
b) a bulk portion;
wherein both the textured surface and/or the bulk portion comprise a polymeric matrix, for promoting active neural cell network growth *in vitro* and/or *ex-vivo.*

2. *In vitro* and/or *ex-vivo* culturing method comprising the steps:
i) providing
a) the substrate as defined in claim 1; and
b) primary cultures developed from dissociated brain cells and organotypic cultures from explanted neural tissue; wherein the primary cultures comprise cells;
wherein the substrate and the cells are in an *in vitro* and/or *ex-vivo* culture media; and
wherein the textured surface with a plurality of protrusions of the substrate (a) is in contact with the surface of one or several cells (b) in the culture media;
ii) culturing the cells to obtain a neural cell network; and
iii) measuring the neural cell network activity.

3. A cultured neural cell network obtainable by the culturing method according to claim 2; wherein the neural cell network is active.

4. Use of the substrate as defined in claim 1
- wherein both the textured surface and/or the bulk portion comprise particles having a mean size of between 1 nm and 1000 µm;
- wherein said particles are homogeneously distributed among the bulk portion and the textured surface; and
- wherein the particles are electrically conductive,
for promoting neural cell network growth *in vitro* and/or *ex-vivo* and/or stimulation *in vitro* and/or *ex-vivo;* preferably for promoting neural cell network 3D growth *in vitro* and/or *ex-vivo* and/or stimulation of electrically excitable cells *in vitro* and/or *ex-vivo.*

5. A probe comprising:
- the substrate as defined in claim 4; and
- an electrical connection comprising a conductive wire;
wherein the probe is optionally implantable.

6. Use of the probe of claim 5 for cell stimulation *in vitro* and/or *ex-vivo.*

7. A stimulation device comprising
- the probe as defined in claim 5; and
- an electrical pulse generator;
wherein the probe and the electrical pulse generator are electrically connected; and wherein the device is optionally implantable.

8. Use of the stimulation device as defined in claim 7 for *in vitro* and/or *ex-vivo* cell stimulation and/or cell neuromodulation; preferably for electrical cell stimulation and/or electrical cell neuromodulation *in vitro* and/or *ex-vivo;* more preferably for electrical neural cell stimulation and/or for electrical neural cell neuromodulation *in vitro* and/or *ex-vivo,* even more preferably for electrical cell growth stimulation and/or for electrical cell growth neuromodulation *in vitro* and/or *ex-vivo.*

9. A monitor device comprising
- the probe as defined in claim 5 or the stimulation device as defined in claim 7; and
- an electrical monitor;
wherein the probe and the electrical monitor are electrically connected.

10. Use of the monitor device of claim 9 for cell monitoring *in vitro* and/or *ex-vivo;* preferably for electrical cell monitoring *in vitro* and/or *ex-vivo;* more preferably for electrical neural cell monitoring *in vitro* and/or *ex-vivo.*

11. A composition obtainable by the method comprising the steps of:
- providing
∘ the probe of claim 5 or the stimulation device of claim 7 or the monitor device of claim 10; and
∘ one or several electrically excitable cells in an *in vitro* and/or *ex-vivo* media;
- contacting the textured surface of the substrate of the probe with the surface of one or several electrically excitable cells; and
- electrically stimulating the one or several electrically excitable cells to obtain a composition.

12. A composition 2 comprising:
- the probe of claim 5 or the stimulation device of claim 7 or the monitor device of claim 9; and
- electrically excitable cells;
wherein the textured surface of the substrate of the probe is in contact with the surface of one or several cells in an *in vitro* and/or *ex-vivo media.*

13. The composition according to anyone of claims 11-12 for use as a medicament.

14. The composition according to anyone of claims 11-12 for use in the treatment of chronic pain, epilepsy, and Parkinson's disease, sacral nerve pelvic disorders or incontinence, spinal cord traumatic disorders and spinal cord ischemic disorders (such as angina or peripheral vascular disease).

15. A kit comprising:
- the probe of claim 5 or the stimulation device of claim 7 or the monitor device of claim 10;
- electrically excitable cells; and
- at least a container;
wherein the textured surface of the substrate of the probe is in contact with the surface of one or several cells in an *in vitro* and/or *ex-vivo media.*
